(19) Europäisches Patentamt
European Patent Office
Office européen des brevets

(11) Veröffentlichungsnummer: **0 128 116**
**B1**

(12) # EUROPÄISCHE PATENTSCHRIFT

(45) Veröffentlichungstag der Patentschrift: **12.08.87**

(21) Anmeldenummer: **84810215.8**

(22) Anmeldetag: **07.05.84**

(51) Int. Cl.⁴: **C 07 D 403/12**, C 07 D 411/12, A 01 N 47/36 // C07D327/06, C07D217/24

(54) **Anellierte N-Phenylsulfonyl-N'-pyrimidinyl- und -triazinylharnstoffe.**

(30) Priorität: **11.05.83 CH 2592/83**

(43) Veröffentlichungstag der Anmeldung: **12.12.84 Patentblatt 84/50**

(45) Bekanntmachung des Hinweises auf die Patenterteilung: **12.08.87 Patentblatt 87/33**

(84) Benannte Vertragsstaaten: **AT BE CH DE FR GB IT LI NL SE**

(56) Entgegenhaltungen:
**EP-A-0 044 807**
**EP-A-0 051 465**
**EP-A-0 099 339**
**EP-A-0 107 979**

(73) Patentinhaber: **CIBA-GEIGY AG, Klybeckstrasse 141, CH-4002 Basel (CH)**

(72) Erfinder: **Ehrenfreund, Josef, Dr., Amselstrasse 11, CH-4123 Allschwil (CH)**
Erfinder: **Föry, Werner, Dr., Benkenstrasse 65, CH-4054 Basel (CH)**

**EP 0 128 116 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

## Beschreibung

Die vorliegende Erfindung betrifft neue, herbizid wirksame und pflanzenwuchsregulierende anellierte N-Phenylsulfonyl-N'-pyrimidinyl- und -triazinyl-harnstoffe, Verfahren zu ihrer Herstellung, sie als Wirkstoffe enthaltende Mittel sowie deren Verwendung zum Bekämpfen von Unkräutern, vor allem selektiv in Nutzpflanzenkulturen oder zum Regulieren und Hemmen des Pflanzenwachstums. Darüberhinaus betrifft die Erfindung auch als Zwischenprodukte hergestellte neue anellierte Phenylsulfonylisocyanate, -thioisocyanate, Phenylsulfonylcarbamate, Phenylsulfonamide sowie Phenylsulfonylchloride.

Die erfindungsgemässen anellierten N-Phenylsulfonyl-N"-pyrimidinyl- und -triazinyl-harnstoffe entsprechen der Formel I

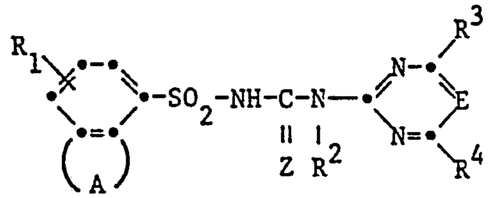

(I),

worin

Z Sauerstoff oder Schwefel,

E Stickstoff oder =CH-,

$R^1$ Wasserstoff, Halogen, Nitro, $C_1$-$C_4$-Alkyl, $C_1$-$C_4$-Halogenalkyl, Cyan oder eine Gruppe -X-$R^5$, -CO-X-$R^6$, -CO-$NR^7R^8$, -SO-$R^9$ oder -$SO_2$-$R^{10}$,

$R^2$ Wasserstoff, $C_1$-$C_4$-Alkyl oder $C_1$-$C_4$-Alkoxy,

$R^3$ $C_1$-$C_4$-Alkyl, $C_1$-$C_4$-Halogenalkyl, $C_1$-$C_4$-Alkoxy, $C_1$-$C_4$-Halogenalkoxy oder $C_3$-$C_6$-Cycloalkyl,

$R^4$ Wasserstoff, Halogen, $C_1$-$C_4$-Halogenalkyl, $C_1$-$C_4$-Alkoxy, $C_1$-$C_4$-Halogenalkoxy, $C_2$-$C_4$-Alkoxyalkoxy, $C_3$-$C_6$-Cycloailkyl oder -$NR^{11}R^{12}$,

$R^5$ $C_3$-$C_5$-Alkinyl oder unsubstituiertes oder durch Halogen oder $C_1$-$C_4$-Alkoxy substituiertes $C_1$-$C_4$-Alkyl oder $C_3$-$C_5$-Alkenyl,

$R^6$ und $R^9$ $C_1$-$C_4$-Alkyl, $C_1$-$C_4$-Halogenalkyl, $C_2$-$C_4$-Alkoxyalkyl, $C_3$-$C_5$-Alkenyl, $C_3$-$C_5$-Alkinyl, Phenyl oder Benzyl,

$R^7$ und $R^8$ unabhängig voneinander Wasserstöff, $C_1$-$C_4$-Alkyl, $C_1$-$C_4$-Halogenalkyl, $C_2$-$C_4$-Alkoxyalkyl, $C_3$-$C_5$-Alkenyl, $C_3$-$C_5$-Alkinyl, Phenyl oder Benzyl,

$R^{10}$ $C_1$-$C_4$-Alkyl, $C_1$-$C_4$-Alkoxy, $C_1$-$C_4$-Halogenalkoxy oder -$NR^{13}$ $R^{14}$

$R^{11}$ und $R^{12}$ unabhängig woneinander Wasserstoff, oder $C_1$-$C_4$-Alkyl,

$R^{13}$ und $R^{14}$ unabhängig voneinander Wasserstoff, $C_1$-$C_4$-Alkyl, $C_1$-$C_4$-Halogenalkyl, $C_2$-$C_4$-Alkoxyalkyl, $C_3$-$C_5$-Alkenyl, $C_3$-$C_5$-Alkinyl, Phenyl oder Benzyl,

X Sauerstoff oder Schwefel bedeuten und

A für eine gegebenenfalls substituierte 4-atomige ungesättigte Brücke, der Formel -CH=CH-Y- steht, worin Y das zweiatomige Brückenglied O-$SO_2$- bedeutet und die

Substituenten der Brücke A ausgewählt sind aus der Gruppe Halogen, Cyan, $C_1$-$C_4$-Alkyl, $C_1$-$C_4$-Halogenalkyl, $C_1$-$C_4$-Alkoxy, $C_1$-$C_4$-Alkylcarbonyl, $C_1$-$C_4$-Alkoxycarbonyl, $C_1$-$C_4$-Alkylthiocarbonyl, Carbamoyl, $C_1$-$C_4$-Alkylaminocarbonyl, Di-$C_1$-$C_4$-alkylaminocarbonyl, $C_1$-$C_4$-Alkylsulfonyl, $C_3$-$C_5$-Alkenyl oder $C_3$-$C_5$-Alkinyl; sowie den Salzen dieser Verbindungen.

Harnstoffverbindungen, Triazinverbindungen und Pyrimidinverbindungen mit herbizider Wirkung sind allgemein bekannt. Kürzlich wurden Sulfonylharnstoffverbindungen mit herbizider und pflanzenwuchsregulierender Wirkung, beispielsweise in den europäischen Patentanmeldungen 44 807, 44 808, 51 465, 99 399 und 107 979 beschrieben.

In den Definitionen ist unter Alkyl geridkettiges oder verzweigtes Alkyl zu verstehen; z. B.: Methyl, Äthyl, n-Propyl, i-Propyl oder die vier isomeren Butyl.

Unter Alkoxy ist zu verstehen: Methoxy, Äthoxy, n-Propyloxy, i-Propyloxy oder die vier isomeren Butyloxyreste, insbesondere aber Methoxy, Äthoxy oder i-Propyloxy.

Beispiele für Alkylthio sind Methylthio, Äthylthio, n-Propylthio, i-Propylthio, n-Butylthio oder n-Pentylthio insbesondere aber Methylthio und Äthylthio.

Beispiele für Alkylsulfinyl sind Methylsulfinyl, Äthylsulfinyl, n-Propylsulfinyl und n-Butylsulfinyl, insbesondere aber Methylsulfinyl und Äthylsulfinyl.

Beispiele für Alkylsulfonyl sind Methylsulfonyl, Äthylsulfonyl oder n-Propylsulfonyl, insbesondere aber Methylsulfonyl und Äthylsulfonyl.

Unter Halogen in den Definitionen sowie als Teil in Halogenalkoxy sind Fluor, Chlor und Brom, vorzugsweise jedoch Fluor und Chlor zu verstehen.

Die Erfindung umfasst ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium,

Magnesium oder Calcium hervorzuheben, insbesondere aber die von Natrium oder Kalium.

Beispiele für zur Salzbildung geeigneten Amine sind primäre, sekundäre und tertiäre aliphatische und aromatische Amine wie Methylamin, Äthylamin, Propylamin, i-Propylamin, die vier isomeren Butylamine, Dimethylamin, Diäthylamin, Diäthanolamin, Dipropylamin, Diisopropylamin, Di-n-butylamin, Pyrrolidin, Piperidin, Morpholin, Trimethylamin, Triäthylamin, Tripropylamin, Chinuclidin, Pyridin, Chinolin und i-Chinolin, insbesondere aber Äthyl- Propyl-, Diäthyl- oder Triäthylamin, vor allem aber iso-Propylamin und Diäthinolamin.

Beispiele für quaternäre Ammoniumbasen sind im allgemeinen die Kationen von Halogenammoniumsalzen, z. B. das Tetrimethylammoniumkation, das Trimethylbenzylammoniumkation, das Triäthylbenzylammoniumkation, das Tetraäthylammoniumkation, das Trimethyläthylammoniumkition, aber auch das Ammoniumkation.

Durch den Phenylring und anellierte 4-atomige Brücke A werden kondensierte heterocyclische ungesättigte Ringsysteme gebildet, in denen das Kettenglied Schwefel in oxidierten Form vorliegt. Somit sind von der Erfindung solche Heterocyclen umfasst, die Sultonfunktion enthalten.

Als Grundtypen der kondensierten Heterocyclen, die aus dem sulfonylsubstituierten Phenylring und der anellierten Brücke A gebildet werden, sind zu nennen:

1,2-Benzoxathiin-S,S-dioxid und 1H-2,1-Benzothiazin-S,S-dioxid.

Unter den Verbindungen der Formel I sind diejenigen bevorzugt, in denen entweder

a) die Substituenten der Brücke A Halogen oder $C_1$-$C_4$-Alkyl sind oder

b) Z Sauerstoff ist oder

c) $R^1$ Wasserstoff oder Halogen ist oder

d) $R^2$ für Wasserstoff steht oder

e) $R^3$ und $R^4$ unabhängig voneinander $C_1$-$C_4$-Alkyl, $C_1$-$C_4$-Alkoxy, Di-$C_1$-$C_4$-alkylamino, $C_1$-$C_4$-Halogenalkoxy oder Halogen bedeuten und zusammen höchstens 4 Kohlenstoffatome enthalten.

Weiter bevorzugte Untergruppen enthalten Verbindungen der Formel I, in denen

Z Sauerstofoff, $R^1$ Wasserstoff oder Halogen, $R^2$ Wasserstoff, und $R^3$ und $R^4$ unabhängig voneinander $C_1$-$C_4$-Alkyl, $C_1$-$C_4$-Alkoxy, Di-$C_1$-$C_4$-alkylamino, $C_1$-$C_4$-Halogenalkoxy oder Halogen, welche zusammen höchstens 4 Kohlenstoffatome enthalten, bedeuten.

Eine besonders bevorzugte Gruppe von Wirkstoffen der Formel I bilden diejenigen Verbindungen, in denen

Z Sauerstoff, $R^1$ Wasserstoff, Halogen oder $C_1$-$C_4$-Alkoxycarbonyl, $R^2$ Wasserstoff, $R^3$ und $R^4$ unabhängig voneinander $C_1$-$C_4$-Alkyl, $C_1$-$C_4$-Alkoxy, Di-$C_1$-$C_4$-alkylamino, $C_1$-$C_4$-Halogenalkoxy oder Halogen, welche zusammen höchstens 4 Kohlenstoffatome enthalten, bedeuten und die Brücke A gegebenenfalls durch Halogen oder $C_1$-$C_4$-Alkyl substituiert ist.

Als bevorzugte Einzelsubstanzen sind zu nennen:

N-(6-Brom-3-methyl-2,2-dioxo-1,2-benzoxathiin-8-ylsulfonyl)-N''-(4-methoxy-6-methyl-1,3,5-triazin-2-yl)-harnstoff, und N-(3-Methyl-2,2-dioxo-1,2-benzoxathiin-8-ylsulfonyl)-N'-(4-methoxy-6-methyl-1,3,5-triazin-2-yl)-harnstoff.

Die Herstellung der Verbindungen der Formel I erfolgt im allgemeinen nach den folgenden Methoden.

Nach einem ersten Verfahren werden die Verbindungen der Formel I erhalten, indem man ein anelliertes Phenylsulfonamid der Formel II

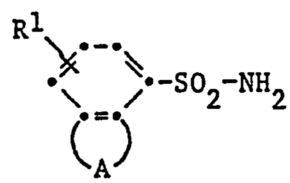

(II) ,

worin $R^1$ und A die unter Formel I gegebene Bedeutung haben, in Gegenwart einer Base mit einem N-Pyrimidinyl- oder -triazinylcarbamat der Formel III

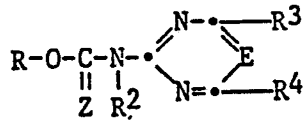

(III) ,

worin

E, $R^2$, $R^3$, $R^4$ und Z die unter Formel I gegeben haben und R für Phenyl, Alkyl oder substituiertes Phenyl steht, umsetzt.

Nach einem zweiten Verfahren gelangt man zu Verbindungen der Formel I, indem man ein anelliertes Phenylsulfonylisocyanat oder -isothiocyanat der Formel IV

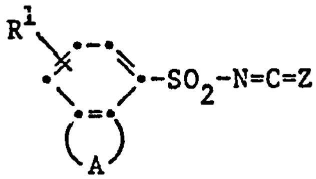

(IV) ,

worin A, $R^1$ und Z die unter Formel I gegebene Bedeutung haben, in Gegenwart einer Base mit einem Aminopyrimidin oder -triazin der Formel V

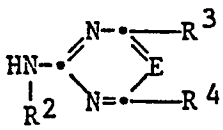

(V) ,

worin
E, $R^2$, $R^3$ und $R^4$ die unter Formel I gegebene Bedeutung haben, umsetzt.

Schliesslich kann man die Verbindungen der Formel I auch erhalten, indem man ein anelliertes N-Phenylsulfonylcarbamat der Formel VI

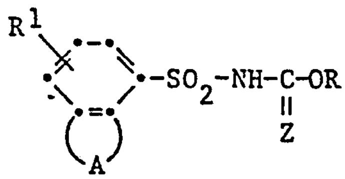

(VI) ,

worin A, $R^1$ und Z die unter Formel I gegebene Bedeutung haben und R für Phenyl, Alkyl oder substituiertes Phenyl steht, mit einem Aminopyrimidin oder -triazin der oben angegebene Formel V umsetzt.

Die erhaltene Harnstoffe der Formel I können gewünschtenfalls mittels Aminen, Alkalimetall- oder Erdalkalimetallhydroxiden oder quaternären Ammoniumbasen in Additionssalze überführt werden. Dieses geschieht beispielsweise durch Umsetzen mit der äquimolaren Menge Base und Verdampfen des Lösungsmittels.

Die Umsetzungen zu Verbindungen der Formel I werden vorteilhafterweise in aprotischen, inerten organischen Lösungsmittel vorgenommen. Solche Lösungsmittel sind Kohlenwasserstoffe wie Benzol, Toluol, Xylol oder Cyclohexan, chlorierte Kohlenwasserstoffe wie Methylenchlorid, Chloroform, Tetrachlorkohlenstoff, oder Chlorbenzol, Äther wie Diäthyläther, Äthylenglykoldimethyläther, Diäthylenglykoldimethyläther, Tetrahydrofuran oder Dioxan, Nitrile wie Acetonitril oder Propionitril, Amide wie Dimethylformamid, Diäthylformamid oder N-Methylpyrrolidinon. Die Reaktionstemperaturen liegen vorzugsweise zwischen -20° und +120°C. Die Umsetzungen der Kupplungsverfahren verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base oder Isocyanat als Reaktionskatalysator verkürzt werden. Als Basen sind insbesondere tertiäre Anine wie Trimethylamin, Triäthylamin, Chinuclidin, 1,4-Diazabicyclo-(2,2,2)-octan, 1,5-Diazabi-cyclo(4,3,0)non-5-en oder 1,5-Diazabicyclo(5,4,0)undec-7-en geeignet. Als Basen können aber auch anorganische Basen wie Hydride wie Natrium- oder Calciumhydrid, Hydroxide wie Natrium- und Kaliumhydroxid, Carbonate wie Natrium- und Kaliumcarbonat oder Hydrogencarbonate wie Kalium- und Natriumhydrogencarbonat verwendet werden.

Die Endprodukte der Formel I können durch Einengen und/oder Verdampfen des Lösungsmittels isoliert und durch Umkristallisieren oder Zerreiben des festen Rückstandes in Lösungsmitteln, in denen sie sich nicht gut lösen, wie Äther, aromatischen Kohlenwasserstoffen oder chlorierten Kohlenwasserstoffen gereinigt werden.

Die Zwischenprodukte der Formeln II, IV und VI sind neu und wurden speziell für die Synthese der Verbindungen der Formel I entwickelt. Sie bilden daher einen Bestandteil der vorliegenden Erfindung.

Die neuen Zwischenprodukte der Formel II können auf verschiedenen Wegen hergestellt werden. So erhält man die Verbindungen der Formel II, indem man Aniline der Formel VII

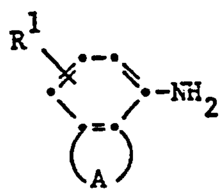

(VII) ,

worin $R^1$ und A die unter Formel I gegebene Bedeutung haben, diazotiert und die Diazogruppe mit Schwefeldioxid in Gegenwart eines Katalysators wie Kupferchlorid in Salzsäure oder Essigsäure austauscht und das entstandene Phenylsulfonylchlorid der Formel X

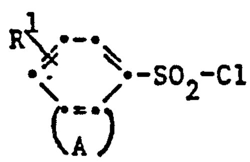

(X) ,

worin A und $R^1$ die unter Formel I angegebene Bedeutung haben, mit Ammoniumhydroxid-Lösung umsetzt. Die als Ausgangsprodukte verwendeten entsprechenden Anilin-Derivate sind bekannt oder nach bekannten Methoden herstellbar.

Ebenso kann man die Verbindungen der Formel II erhalten, indem man eine Phenylsulfonsäure der Formel VIII

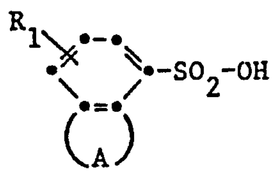

(VIII),

worin $R^1$ und A die unter Formel I gegebene Bedeutung haben, durch Behandeln mit einem Chlorierungsmittel wie $PCl_5$, $POCl_3$, $COCl_2$ oder $SOCl_2$ in das entsprechende Phenylsulfonylchlorid der Formel X überführt und dieses mit Ammoniumhydroxid-Lösung umsetzt.

Weiter kann man die Verbindungen der Formel II erhalten, indem man einen Benzylthioäther der Formel IX

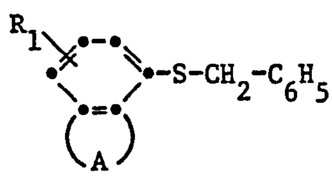

(IX)

worin $R^1$ und A die unter Formel I gegebene Bedeutung haben, durch Behandeln mit Chlor in das entsprechende Phenylsulfonylchlorid der Formel X überführt und dieses mit Ammoniumhydroxid-Lösung umsetzt.

Die ebenfalls neuen Phenylsulfonylisocyanate der Formel IV können beispielsweise durch Umsetzungen der Sulfonamide der Formel II mit Phosgen in Anwesenheit von Butylisocyanat in einem chlorierten Kohlenwasserstoff als Lösungsmittel, bei Rückflusstemperatur erhalten werden. Ähnliche Darstellungen sind in "Neuere Methoden der präparativen organischen Chemie", Band VI, 211-229, Verlag Chemie, Weinheim, 1970, beschrieben.

Die Isothiocyanate der Formel IV werden durch Behandlung der Sulfonamide der Formel II mit Schwefelkohlenstoff und Kaliumhydroxid und anschliessende Umsetzung des Dikaliumsalzes mit Phosgen erhalten. Solche Verfahren sind in Arch. Pharm. 299, 174 (1966) beschrieben.

Die N-Phenylsulfonylcarbamate der Formel VI werden durch Umsetzung der Sulfonamide der Formel II mit Diphenylcarbonat in Gegenwart einer Base erhalten. Ähnliche Verfahren sind in der japanischen Patentschrift 61 169 erwähnt.

Die ebenfalls neuen anellierten Phenylsulfonylchloride der Formel X wurden ebenfalls speziell für die Synthese der Wirkstoffe der Formel I entwickelt. Sie bilden daher einen weiteren Aspekt der vorliegenden Erfindung.

Die als Ausgangsmaterialien verwendeten Aminopyrimidine und -triazine der Formel V sowie entsprechende Phenylcarbamate der Formel III sind entweder seit langem bekannt oder in der europäischen Patentanmeldung

Nr. 70 804 beschrieben oder sie lassen sich nach bekannten Methoden aus dort beschriebenen Verbindungen erhalten.

Die Endprodukte können durch Einengen und/oder Verdampfen des Lösungsmittels isoliert und durch Umkristallisieren oder Zerreiben des festen Rückstandes in Lösungsmitteln in denen sie sich nicht gut lösen, wie Äther, aromatischen Kohlenwasserstoffen oder chlorierten Kohlenwasserstoffen gereinigt werden.

Die Wirkstoffe der Formel I sind stabile Verbindungen. Ihre Handhabung bedarf keine vorsorglicher Massnahmen.

Bei geringeren Aufwandmengen zeichnen sich die Verbindungen der Formel I durch gute selektivwuchshemmende und selektiv-herbizide Eigenschaften aus, die sie ausgezeichnet zum Einsatz in Kulturen von Nutzpflanzen, insbesondere in Getreide, Baumwolle, Soja, Mais und Reis, befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei - im Vergleich zu anderen Herbiziden und Wuchsregulatoren - sehr geringen Aufwandmengen.

Die Verbindungen der Formel I haben ausserdem starke pflanzenwuchsregulierende, insbesondere pflanzenwuchshemmende, Eigenschaften. Es werden sowohl Monokotyledonen als auch Dikotyledonen in ihrem Wachstum beeinträchtigt.

So können z. B. die in der Landwirtschaft in tropischen Gegenden häufig als "cover crops" (Bodenbedecker) angeflanzen Leguminosen durch die Verbindungen der Formel I in ihrem Wachstum selektiv gehemmt werden, so dass zwar die Bodenerosion zwischen den Kulturpflanzen verhindert wird, die "cover crops" jedoch nicht zur Konkurrenz für die Kultur werden können.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruhrt darauf, dass die Nährstoffe in stärkerem Masse der Blüten- und Fruchtbildung zugute kommen, während das vegetative Wachstum eingeschränkt wird.

Bei grösseren Aufwandmengen werden alle getesteten Pflanzen in ihrer Entwicklung so geschädigt, dass sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe bevorzugt die Fraktionen $C_8$ bis $C_{12}$ wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen:

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren

Fettsäuren ($C_{10}$-$C_{22}$), wie z. B. die Na- oder K-Salze der Öl- oder Stearinsäure, oder von natürlichen Fettsäuregemischen, die z. B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z. B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Äthylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 10 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Äthylsulfate vor, z. B. das Stearyltrimethylammoniumchlorid oder das Benzyldi(2-chloräthyl)-äthyl-ammoniumbromid.

Die in der Formulierungstechnik gebräuchlichen Tenside sind u.a. in folgenden Publikationen beschrieben:
"Mc Cutcheon's Detergents and Emulsifiers Annual", MC Publishing Corp., Ridgewood, New Jersey, 1981;
H. Stache, "Tensid-Taschenbuch", 2. Aufl., C. Hanser Verlag, München, Wien, 1981;
M. and J. Ash. "Encyclopedia of Surfactants", Vol. I-III, Chemical Publishing Co., New York, 1980-1981.

Die pestiziden Zubereitungen enthalten in der Regel 0,1 bis 95 %, insbesondere 0,1 bis 80 %, Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent)

Emulgierbare Konzentrate:

| | |
|---|---|
| Aktiver Wirkstoff: | 1 bis 20 %, bevorzugt 5 bis 10 % |
| oberflächenaktives Mittel: | 5 bis 30 %, vorzugsweise 10 bis 20 % |
| flüssiges Trägermittel: | 50 bis 94 %, vorzugsweise 70 bis 85 %. |

Stäube

| | |
|---|---|
| Aktiver Wirkstoff: | 0,1 bis 10 %, vorzugsweise 0,1 bis 1 % |
| festes Trägermittel: | 99,9 bis 90 %, vorzugsweise 99,9 bis 99 %. |

Suspensions-Konzentrate:

| | |
|---|---|
| Aktiver Wirkstoff: | 5 bis 75 %, vorzugsweise 10 bis 50 % |
| Wasser: | 94 bis 25 %, vorzugsweise 90 bis 30 % |
| oberflächenaktives Mittel: | 1 bis 40 %, vorzugsweise 2 bis 30 %. |

Benetzbare Pulver:

| | |
|---|---|
| Aktiver Wirkstoff: | 0,5 bis 90 %, vorzugsweise 1 bis 80 % |
| oberflächenaktives Mittel: | 0,5 bis 20 %, vorzugsweise 1 bis 15 % |
| festes Trägermittel: | 5 bis 95 %, vorzugsweise 15 bis 90 %. |

| | |
|---|---|
| Aktiver Wirkstoff: | 0,5 bis 30 %, vorzugsweise 3 bis 15 % |
| festes Trägermittel: | 99,5 bis 70 %, vorzugsweise 97 bis 85 %. |

Während als Handelsware eher konzentrierte Mittel bevorzugt werden, verwendet der Endverbraucher in der

Regel verdünnte Mittel. Die Anwendungsformen können bis hinab zu 0,001 % an Wirkstoff verdünnt werden. Die Aufwandmengen betragen in der Regel 0,01 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

**Herstellungsbeispiele:**

**Beispiel H1: N-(6-Brom-2,2-di-oxo-1,2-benzoxathi-in-8-yl-sulfonyl)-N'-(4,6-dimethoxy-pyrimidin-2-yl)-harnstoff**

a) 6-Brom-3,4-dihydro-4-hydroxy-2,2-dioxo-1,2-benzoxathiin. 24,2 g Salicylaldehyd werden in 100 ml absolutem Pyridin gelöst und bei einer Temperatur von 0°C tropfenweise mit 17 ml Methylsulfonsäurechlorid versetzt. Nachdem das Reaktionsgemisch für 5 Stunden bei 20-25°C gerührt worden ist, wird es in ein Gemisch von Eis und 10 %iger Salzsäure eingegossen. Diese Mischung wird mit Äther extrahiert. Die organischen Phasen werden über Natriumsulfat getrocknet und eingedampft. Man erhält so 30 g 5-Brom-2-methylsulfonyloxybenzaldehyd. Dieses Zwischenprodukt wird in 100 ml Methylenchlorid gelöst und bei einer Temperatur von 0°C tropfenweise mit 16 ml 1,5-Diazabicyclo(5,4,0)undec-5-en versetzt. Die Mischung wird anschliessend für 30 Minuten gerührt und in ein Gemisch von Eis und 10 %iger Salzsäure eingegossen. Durch Extraktion mit Äther, Trocknen und Verdampfen der organischen Phase erhält man 23,0 g 6-Brom-3,4-dihydro-4-hydroxy-2,2-dioxo-1,2-benzoxathiin.

b) 6-Brom-2,2-dioxo-1,2-benzoxathiin.

Die unter Abschnitt a) erhaltenen 23,0 g 6-Brom-3,4-dihydro-4-hydroxy-2,2-dioxo-1,2-benzoxathiin werden ohne weitere Reinigung in 50 ml Pyridin gelöst. Anschliessend wird die Lösung auf 0°C abgekühlt und tropfenweise mit 16,0 g Phosphoroxytrichlorid versetzt. Nachdem die Mischung für 3 Stunden bei einer Temperatur von 20-25°C gerührt worden ist, wird es vorsichtig im Eiswasser eingegossen, wobei das Rohprodukt ausfällt. Der Niederschlag wird abgetrennt und aus Äthanol umkristallisiert. Man erhält so 17,5 g (82 % d. Th.) 6-Brom-2,2-dioxo-1,2-benzoxathiin, Smp. 140-141,5°C.

c) 6-Brom-8-chlorsulfonyl-2,2-dioxo-1,2-benzoxathiin.

6,0 g 6-Brom-2,2-dioxo-1,2-benzoxathiin werden mit 22,0 g Chlorsulfonsäure für 1 Stunde auf 60°C erhitzt. Anschliessend wird das Gemisch im Eiswasser eingegossen. Das ausgefallene 6-Brom-8-chlorsulfonyl-2,2-dioxo-1,2-benzoxathiin wird abgetrennt, mit Wasser gewaschen und getrocknet.

d) 6-Brom-8-sulfamoyl-2,2-dioxo-1,2-benzoxathiin.

Das unter c) erhaltene Kristallisat wird in möglichst wenig Tetrahydrofuran gelöst und tropfenweise in 20 ml 25 %iger Ammoniaklösung eingetragen. Nach dem die Reaktionsmischung für 30 Minuten gerührt werden ist wird sie auf ein Gemisch aus Eis und 10 %iger Salzsäure gegossen. Die Extraktion dieser wässrigen Mischung mit Äther ergibt 0,3 g 6-Brom-8-sulfamoyl-2,2-dioxo-1,2-benzoxathiin, Smp. 224-228°C (Zersetzung unter Schwarzfärbung).

e) N-(6-Brom-2,2-dioxo-1,2-benzoxathiin-8-yl-sulfonyl)-N'-(4,6-dimethoxy-pyrimidin-2-yl)-harnstoff.

0,3 g 6-Brom-8-sulfamoyl-2,2-dioxo-1,2-benzoxathiin werden in 10 ml Acetonitril gelöst. Diese Lösung wird mit 0,25 g N-(4,6-Dimethoxy-pyrimidin-2-yl)-phenylcarbamat und 0,15 g 1,5-Diaza-bicyclo(5.4.0)undec-5-en versetzt und anschliessend für 2 Stunden bei 20-25°C gerührt. Danach wird das Gemisch mit 5 %iger Salzsäure angesäuert und mit Wasser verdünnt. Das ausfallende Öl wird mit Äthylacetat extrahiert, die vereinigten organischen Phasen werden mit Wasser gewaschen, getrocknet und eingedampft. Aus dem öligen Rückstand erhält man durch Kristallisation aus Äther 0,3 g N-(6-Brom-2,2-dioxo-1,2-benzoxathiin-8-yl-sulfonyl)-N'-(4,6-dimethoxy-pyrimidin-2-yl)-harnstoff, Smp. 204-209°C (Zers.).

**Beispiel H2: 3-Methyl-2,2-dioxo-1,2-benzoxathiin-8-yl-sulfonylisocyanat.**

a) N-(3-Methyl-2,2-dioxo-1,2-benzoxathiin-8-yl-sulfonyl)-N'-methyl-harnstoff.

In eine Lösung von 5,5 g 3-Methyl-8-sulfamoyl-2,2-dioxo-1,2-benzoxathiin und 3,2 g 1,5-Diazabicyclo(5,4,0)undec-5-en wird bei 0°C tropfenweise mit 1,15 g Methylisocyanat versetzt. Die Reaktionsmischung wird bei einer Temperatur von 20-25°C für 2 Stunden gerührt, anschliessend mit Wasser verdünnt, mit 10 ml 5 %iger Natriumcarbonatlösung versetzt und filtriert. Beim Ansäuern des Filtrats fallen 5,4 g N-(3-Methyl-2,2-dioxo-1,2-benzoxathiin-8-yl-sulfonyl)-N'-methyl-harnstoff aus. Dieser Niederschlag wird abgetrennt und getrocknet.

b) 3-Methyl-2,2-dioxo-1,2-benzoxathiin-8-yl-sulfonylisocyanat.

5,0 g N-(3-Methyl-2,2-dioxo-1,2-benzoxathiin-8-yl-sulfonyl)-N'-methyl-harnstoff werden in 150 ml absolutem Chlorbenzol dispergiert und bei einer Temperatur von 130°C mit Phosgen gesättigt. Beim Einleiten des Phosgens entsteht eine klare Lösung. Anschliessend wird das Lösungsmittel im Vakuum unter Feuchtigkeitsausschluss abdestilliert. Das als öliger Rückstand anfallende 3-Methyl-2,2-dioxo-1,2-benzoxathiin-8-yl-sulfonyl-isocyanat kann ohne weitere Reinigung zur Darstellung der erfindungsgemässen Pyrimidinyl- und Triazinylharnstoffe der Formel I verwendet werden.

In analoger Weise erhält man die in dem angeschlossenen Tabellen aufgelisteten Zwischen- und Endprodukte.

**Tabelle 1:**

$Q - SO_2 - NH_2$

| Verb. Nr. | Q | Abkürzung für Q | phys. Daten |
|---|---|---|---|
| 1.1 | 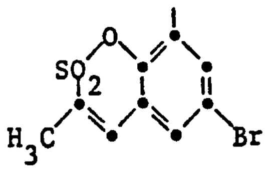 | Q 1 | Smp. 253-255 °C |
| 1.2 | 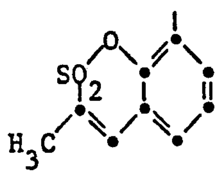 | Q 2 | Smp. 205-206° C |
| 1.3 | 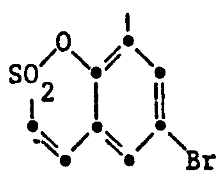 | Q 3 | |

**Tabelle 2:**

$Q - SO_2 - Cl$

| Verb.Nr. | Q | phys. Daten |
|---|---|---|
| 2.1 | Q 1 | |
| 2.2 | Q 2 | |
| 2.3 | Q 3 | |

**Tabelle 3:**

$Q - SO_2 - N = C = O$

| Verb Nr. | Q | phys. Daten |
|---|---|---|
| 3,1 | Q 1 | Öl |
| 3.2 | Q 2 | Öl |
| 3.3 | Q 3 | Öl |

**Tabelle 4:**

$Q - SO_2 - NH - CO - T$

| Verb.Nr | Q | T | phys. Daten |
|---|---|---|---|
| 4.1 | Q 1 | $OC_6H_5$ | |
| 4.2 | Q 2 | $OC_6H_5$ | |
| 4.3 | Q 3 | $OC_6H_5$ | |
| 4.4 | Q 1 | $OCH_3$ | |
| 4.5 | Q 2 | $OCH_3$ | |
| 4.6 | Q 3 | $OCH_3$ | |
| 4.7 | Q 1 | $OC_2H_5$ | |
| 4.8 | Q 2 | $OC_2H_5$ | |
| 4.9 | Q 3 | $OC_2H_5$ | |
| 4.10 | Q 1 | $-NHCH_3$ | |
| 4.11 | Q 2 | $-NHCH_3$ | |
| 4.12 | Q 3 | $-NHCH_3$ | |

**Tabelle 5**

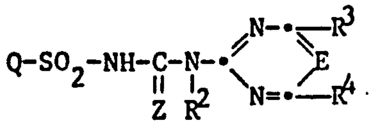

Q hat die in der Tabelle 1 gegebenen Bedeutungen für den anellierten Phenylring.

| Verb. Nr. | Q | $R^2$ | $R^3$ | $R^4$ | Z | E | Smp. [°C] |
|---|---|---|---|---|---|---|---|
| 5.1 | Q 1 | H | $OCH_3$ | $CH_3$ | 0 | N | 196-198 |
| 5.2 | Q 1 | H | $OCH_3$ | $OCH_3$ | 0 | N | |
| 5.3 | Q 1 | H | $OCH_3$ | $-N(CH_3)_2$ | 0 | N | |
| 5.4 | Q 1 | H | $OCH_3$ | $OCH_2-CF_3$ | 0 | N | |
| 5.5 | Q 1 | H | $CH_3$ | $C_2H_5$ | 0 | N | |
| 5.6 | Q 1 | H | $CH_3$ | $CH_3$ | 0 | CH | |
| 5.7 | Q 1 | H | $CH_3$ | $OCH_3$ | 0 | CH | |
| 5.8 | Q 1 | H | $OCH_3$ | $OCH_3$ | 0 | CH | |
| 5.9 | Q 1 | H | $CH_3$ | $OCHF_2$ | 0 | CH | |
| 5.10 | Q 2 | H | $CH_3$ | $OCH_3$ | 0 | N | 203-205 |
| 5.11 | Q 2 | H | $OCH_3$ | $OCH_3$ | 0 | N | |
| 5.12 | Q 2 | H | $OCH_3$ | $-N(CH_3)_2$ | 0 | N | |
| 5.13 | Q 2 | H | $CH_3$ | $C_2H_5$ | 0 | N | |
| 5.14 | Q 2 | H | $OCH_3$ | $C_2H_5$ | 0 | N | |
| 5.15 | Q 2 | H | $OC_2H_5$ | $C_2H_5$ | 0 | N | |
| 5.16 | Q 2 | H | $OCH_3$ | $OCH_2-CF_3$ | 0 | N | |
| 5.17 | Q 2 | H | $CH_3$ | $OCH_2-CF_3$ | 0 | N | |
| 5.18 | Q 2 | H | $OCH_3$ | $OC_3H_7-i$ | 0 | N | |
| 5.19 | Q 2 | H | $SCH_3$ | $OCH_3$ | 0 | N | |
| 5.20 | Q 2 | H | $OCH_3$ | $-NHCH_3$ | 0 | N | |
| 5.21 | Q 2 | H | $CH_3$ | $CH_3$ | 0 | N | |
| 5.22 | Q 2 | H | $CH_3$ | $CH_3$ | 0 | CH | |
| 5.23 | Q 2 | H | $OCH_3$ | $CH_3$ | 0 | CH | |
| 5.24 | Q 2 | H | $OCH_3$ | $OCH_3$ | 0 | CH | |
| 5.25 | Q 2 | H | $CH_3$ | $OCH_2-CF_3$ | 0 | CH | |
| 5.26 | Q 2 | H | $CH_3$ | $OC_2H_5$ | 0 | CH | |
| 5.27 | Q 2 | H | $OCH_3$ | Cl | 0 | CH | |
| 5.28 | Q 2 | H | $OCH_3$ | $SCHF_2$ | 0 | CH | |
| 5.29 | Q 2 | H | $OCH_3$ | $-N(CH_3)_2$ | 0 | CH | |
| 5.30 | Q 2 | H | $OCH_3$ | $-NHCH_3$ | 0 | CH | |
| 5.31 | Q 2 | H | $OCH_3$ | $CH_2F$ | 0 | CH | |
| 5.32 | Q 2 | H | $OCH_3$ | $CF_3$ | 0 | CH | |
| 5.33 | Q 2 | H | $OCH_3$ | $SCH_3$ | 0 | CH | |
| 5.34 | Q 2 | H | $OCH_3$ | $OCH_2-CF_3$ | 0 | CH | |
| 5.35 | Q 2 | H | $OCH_3$ | $-CH_2-OCH_3$ | 0 | CH | |
| 5.36 | Q 2 | H | $OCH_3$ | $-CH_2-OC_2H_5$ | 0 | CH | |
| 5.37 | Q 3 | H | $OCH_3$ | $OCH_3$ | 0 | CH | 204-209 |

**Formulierungsbeispiele:**

**Beispiel F1: Formulierungsbeispiele für Wirkstoffe der Formel I (% = Gewichtsprozent)**

a) Spritzpulver

| Spritzpulver | a) | b) | c) |
|---|---|---|---|
| Wirkstoff | 20 % | 60 % | 0,5 % |
| Na-Ligninsulfonat | 5 % | 5 % | 5% |
| Na-Laurylsulfat | 3 % | - | - |
| Na-Diisobutylnaphthalinsulfonat | - | 6 % | 6% |
| Octylphenolpolyäthylenglykoläther (7-8 MolAeO) | - | 2 % | 2% |
| Hochdisperse Kieselsäure | 5 % | 27 % | 27% |
| Kaolin | 67 % | - | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

b) Emulsions-Konzentrat

| Emulsions-Konzentrat | a) | b) |
|---|---|---|
| Wirkstoff | 10 % | 1 % |
| Octylphenolpolyäthylenglykoläther (4-5 Mol AeO) | 3 % | 3 % |
| Ca-Dodecylbenzolsulfonat | 3 % | 3 % |
| Ricinusölpolyglykoläther (36 MolAeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

c) Stäubemittel

| Stäubemittel | a) | b) |
|---|---|---|
| Wirkstoff | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

d) Extruder Granulat

| Extruder Granulat | a) | b) |
|---|---|---|
| Wirkstoff | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| Umhüllungs-Granulat | |
|---|---|
| Wirkstoff | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat

| | a) | b) |
|---|---|---|
| Wirkstoff | 40% | 5% |
| Äthylenglykol | 10% | 10% |
| Nonylphenolpolyäthylenglykoläther (15 Mol AeO) | 6% | 1% |
| Na-Ligninsulfonat | 10% | 5% |
| Carboxymethylcellulose | 1% | 1% |
| 37 %ige wässrige Formaldehyd-Lösung | 0,2 % | 0,2 % |
| Silikonöl in Form einer 75 %igen wässrigen Emulsion | 0,8 % | 0,8 % |
| Wasser | 32% | 77% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) Salzlösung

| | |
|---|---|
| Wirkstoff | 5 % |
| Isopropylamin | 1 % |
| Octylphenolpolyäthylenglykoläther (78Mol AeO) | 3 % |
| Wasser | 91 % |

**Biologische Beispiele:**

**Beispiel B1: Herbizidwirkung vor dem Auflaufen der Pflanzen**

Kunststofftöpfe werden mit expandiertem Vermiculit (Dichte: 0,135 $g/cm^3$, Wasserabsorptionsvermögen: 0,565 l/l) gefüllt. Nach dem Sättigen des nicht adsorptiven Vermiculits mit einer wässrigen Wirkstoffemulsion in deionisiertem Wasser, die die Wirkstoffe in einer Konzentration von 70,8 ppm enthält, werden Samen der folgenden Pflanzen auf die Oberfläche gesät: Nasturtium officinalis, Agrostis tenuis, Stellaria media und Digitaria sanguinalis. Die Versuchsgefässe werden anschliessend in einer Klimakammer bei 20°C, einer Beleuchtung von ca. 20 kLux und einer relativen Luftfeuchtigkeit von 70 % gehalten. Während der Keimphase von 4 bis 5 Tagen werden die Töpfe zur Erhöhung der örtlichen Luftfeuchtigkeit mit lichtdurchlässigem Material abgedeckt und mit deionisiertem Wasser gegossen. Nach dem 5. Tag wird dem Giesswasser 0,5 % eines handelsüblichen Flüssigdüngers (®Greenzit,ex Ciba-Geigy) zugesetzt. 12 Tage nach der Aussaat wird der Versuch ausgewertet und die Wirkung auf die Versuchspflanzen nach dem folgenden Maßstab bewertet:

1: Pflanze nicht gekeimt oder total abgestorben
2-3: sehr starke Wirkung
4-6: mittlere Wirkung
7-8: schwache Wirkung
9: keine Wirkung (wie unbehandelte Kontrolle).

pre-emergente Wirkung:
Konzentration der Wirkstoffemulsion: 70,8 ppm

| Testpflanze / Wirkstoff Nr. | Nasturtium | Stellaria | Agrostis | Digitaria |
|---|---|---|---|---|
| 5.1 | 1 | 1 | 1 | 2 |
| 5.10 | 1 | 1 | 1 | 1 |

**Beispiel B2: Wuchshemmung bei tropischen Bodenbedecker-Leguminosen (cover crops).**

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70 % relativer Luftfeuchtigkeit und 6000 lux Kunstlicht, pro Tag 14 Stunden, bei Temperaturen von 27° bei Tag und 21°C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Formel I behandelten Pflanzen eine deutliche Reduktion des Neuzuwachses (weniger als 20 %

des Neuzuwachses bei unbehandelten Kontrollpflanzen), ohne dass dabei die Versuchspflanzen geschädigt wurden.

**Beispiel B3: Wuchsregulierung an Sojabohnen**

In Kunststoffbehältern mit einem Erde-Torf-Sandgemisch im Verhältnis 6:3:1 werden Sojabohnen der Sorte "Hark" angesät und in eine Klimakammer gegeben. Durch optimale Temperaturwahl, Beleuchtung, Düngerzugabe und Bewässerung entwickeln sich die Pflanzen nach ca. 5 Wochen bis zum 5-6 Trifolia-Blattstadium. Zu diesem Zeitpunkt werden die Pflanzen mit der wässrigen Brühe eines Wirkstoffs der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die erfindungsgemässen Wirkstoffe der Formel I eine merkliche Erhöhung der Anzahl und des Gewichts der Schoten im Haupttrieb.

**Beispiel B4: Wuchshemmung bei Getreide**

In Kunststofftöpfen mit sterilisierter Erde werden in Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Formel I besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zu unbehandelten Kontrollen eine Verringerung des Neuzuwachses (60-90 % der Kontrolle), sowie teilweise eine Zunahme der Stengeldurchmesser auf.

**Beispiel B5: Wuchshemmung bei Gräsern**

In Kunststoffschalen mit Erde-Torf-Sand-Gemisch (6:3:1) werden die Gräser Lolium perenne, Poa pratensis, Festuca ovina, Dactylis glomerate und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge beträgt umgerechnet bis zu 100 g Aktivsubstanz pro Hektar. 21 Tage nach Applikation wird das Wachstum der Gräser beurteilt.

Die Verbindungen der Formel I bewirken eine Reduzierung des Neuzuwachses von um 10-30 % im Vergleich zur unbehandelten Kontrolle.

**Patentansprüche**

für die Vertragsstaaten: BE, CH, LI, DE, FR, GB, IT, NL, SE

1. Anellierte N-Phenylsulfonyl-N'-pyrimidinyl- und -triazinylharnstoffe der Formel I

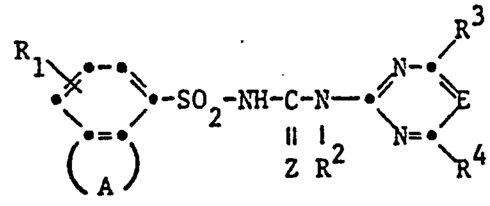

(I),

worin

Z Sauerstoff oder Schwefel,

E Stickstoff oder =CH-,

$R^1$ Wasserstoff, Halogen, Nitro, $C_1$-$C_4$-Alkyl, $C_1$-$C_4$-Halogenalkyl, Cyan oder eine Gruppe -X-$R^5$, -CO-X-$R^6$, -CO-N$R^7R^8$, -SO-$R^9$ oder -$SO_2$2-$R^{10}$,

$R^2$ Wasserstoff, $C_1$-$C_4$-Alkyl oder $C_1$-$C_4$-Alkoxy,

$R^3$ $C_1$-$C_4$-Alkyl, $C_1$-$C_4$-Halogenalkyl, $C_1$-$C_4$-Alkoxy, $C_1$-$C_4$-Halogen-alkoxy oder $C_3$-$C_6$-Cycloalkyl,

$R^4$ Wasserstoff, Halogen, $C_1$-$C_4$-Halogenalkyl, $C_1$-$C_4$-Alkoxy, $C_1$-$C_4$-Halogenalkoxy, $C_2$-$C_4$-Alkoxyalkoxy, $C_3$-$C_6$-Cycloalkyl oder $-NR^{11}R^{12}$,

$R^5$ $C_3$-$C_5$-Alkinyl oder unsubstituiertes oder durch Halogen oder $C_1$-$C_4$-Alkoxy substituiertes $C_1$-$C_4$-Alkyl oder $C_3$-$C_5$-Alkenyl,

$R^6$ und $R^9$ $C_1$-$C_4$-Alkyl, $C_1$-$C_4$-Halogenalkyl, $C_2$-$C_4$-Alkoxyalkyl, $C_3$-$C_5$-Alkenyl, $C_3$-$C_5$-Alkinyl, Phenyl oder Benzyl,

$R^7$ und $R^8$ unabhängig voneinander Wasserstoff, $C_1$-$C_4$-Alkyl, $C_1$-$C_4$-Halogenalkyl, $C_2$-$C_4$-Alkoxyalkyl, $C_3$-$C_5$-Alkenyl, $C_3$-$C_5$-Alkinyl, Phenyl oder Benzyl,

$R^{10}$ $C_1$-$C_4$-Alkyl, $C_1$-$C_4$-Alkoxy, $C_1$-$C_4$-Halogenalkoxy oder $-NR^{13}R^{14}$,

$R^{11}$ und $R^{12}$ unabhängig voneinander Wasserstoff oder $C_1$-$C_4$-Alkyl,

$R^{13}$ und $R^{14}$ unabhängig voneinander Wasserstoff, $C_1$-$C_4$-Alkyl, $C_1$-$C_4$-Halogenalkyl, $C_2$-$C_4$-Alkoxyalkyl, $C_3$-$C_5$-Alkenyl, $C_3$-$C_5$-Alkinyl, Phenyl oder Benzyl,

X Sauerstoff oder Schwefel bedeuten und

A für eine gegebenenfalls substituierte 4-atomige ungesättigte Brücke der Formel -CH=CH-Y- steht, worin Y das zweiatomige Brückenglied $-O-SO_2$ bedeutet und die Substituenten der Brücke A ausgewählt sind aus der Gruppe Halogen, Cyan, $C_1$-$C_4$-Alkyl, $C_1$-$C_4$-Halogenalkyl $C_1$-$C_4$-Alkoxy, $C_1$-$C_4$-Alkyl-carbonyl, $C_1$-$C_4$-Alkoxycarbonyl, $C_1$-$C_4$-Alkylthiocarbonyl, Carbamoyl, $C_1$-$C_4$-Alkylaminocarbonyl, Di-$C_1$-$C_4$-alkylaminocarbonyl, $C_1$-$C_4$-Alkyl-sulfonyl, $C_3$-$C_5$-Alkenyl oder $C_3$-$C_5$, Alkinyl; sowie die Salze dieser Verbindungen.

2. Verbindungen gemäss Anspruch 1, dadurch gekennzeichnet, dass die Substituenten der Brücke A Halogen oder $C_1$-$C_4$-Alkyl sind.

3. Verbindungen gemäss Anspruch 1 dadurch gekennzeichnet dass Z Sauerstoff bedeutet.

4. Verbindungen gemäss Anspruch 1, dadurch gekennzeichnet, dass $R^1$ Wasserstoff oder Halogen bedeutet.

5. Verbindungen gemäss Anspruch 1, dadurch gekennzeichnet, dass $R^2$ für Wasserstoff steht.

6. Verbindungen gemäss Anspruch 1, dadurch gekennzeichnet, dass $R^3$ und $R^4$ unabhängig voneinander $C_1$-$C_4$-Alkyl, $C_1$-$C_4$-Alkoxy, Di-$C_1$-$C_4$-al-kylamino, $C_1$-$C_4$-Halogenalkoxy oder Halogen bedeuten und zusammen höchstens 4 Kohlenstoffatome enthalten.

7. Verbindungen gemäss Anspruch 1, dadurch gekennzeichnet, dass Z Sauerstoff, $R_1$ Wasserstoff oder Halogen, $R^2$ Wasserstoff und $R^3$ und $R^4$ unabhängig voneinander $C_1$-$C_4$-Alkyl $C_1$-$C_4$-Alkoxy, Di-$C_1$-$C_4$-alkylamino, $C_1$-$C_4$-Halogenalkoxy oder Halogen, welche zusammen höchstens 4 Kohlenstoffatome enthalten, bedeuten.

8. Verbindungen gemäss Anspruch 1, dadurch gekennzeichnet, dass Z Sauerstoff, $R^1$ Wasserstoff, Halogen oder $C_1$-$C_4$-Alkoxycarbonyl, $R^2$ Wasserstoff, $R^3$ und $R^4$ unabhängig voneinander $C_1$-$C_4$-Alkyl, $C_1$-$C_4$-Alkoxy, Di-$C_1$-$C_4$-alkyl-amino, $C_1$-$C_4$-Halogenalkoxy oder Halogen, welche zusammen höchstens 4 Kohlenstoffatome enthalten, bedeuten und die Brücke A gegebenenfalls durch Halogen oder $C_1$-$C_4$-Alkyl substituiert ist.

9. N-(3-Methyl-2,2-dioxo-1,2-benzoxathiin-8-ylsulfonyl)-N'-(4-methoxy-6-methyl-1,3,5-triazin-2-yl)-harnstoff gemäss Anspruch 1.

10. N-(6-Brom-3-methyl-2,2-dioxo-1,2-benzoxathiin-8-ylsulfonyl)-N'-(4-methoxy-6-methyl-1,3,5-triazin-2-yl)-harnstoff gemäss Anspruch 1.

11. Anellierte Phenylsulfonamide der allgemeinen Formel II

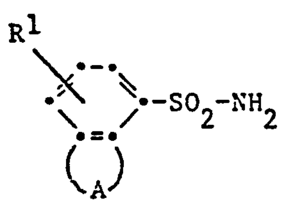

(II)

worin $R^1$ und A die unter Formel I im Anspruch 1 gegebene Bedeutung haben.

12. Anellierte Phenylsulfonylisocyanate und -isothiocyanate der allgemeinen Formel IV

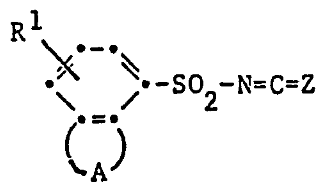

(IV) ,

worin A, $R^1$ und Z die unter Formel I im Anspruch 1 gegebene Bedeutung haben.

13. Anellierte Phenylsulfonylcarbamate der allgemeinen Formel VI

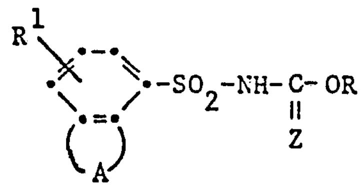

(VI) ,

worin A, $R^1$ und Z die unter Formel I im Anspruch 1 gegebene Bedeutung haben und R für Phenyl, Alkyl oder substituiertes Phenyl steht.

14. Verfahren zur Herstellung der Verbindungen der Formel I, gemäss Anspruch 1 dadurch gekennzeichnet dass man ein anelliertes Phenylsulfonamid der Formel II

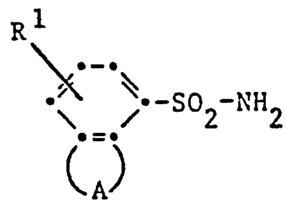

(II)

worin $R^1$ und A die unter Formel I gegebene Bedeutung haben, in Gegenwart einer Base mit einem N-Pyrimidinyl- oder -triazinylcarbamat der Formel III

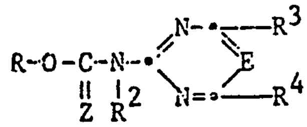

(III)

worin E, $R^2$, $R^3$, $R^4$ und Z die unter Formel I gegebene Bedeutung haben und R für Phenyl, Alkyl oder substituiertes Phenyl steht, umsetzt und gegebenenfalls in die Salze überführt.

15. Verfahren zur Herstellung der Verbindungen der Formel I gemäss Anspruch 1, dadurch gekennzeichnet, dass man ein anelliertes Phenylsulfonylisocyanat oder -isothiocyanat der Formel IV

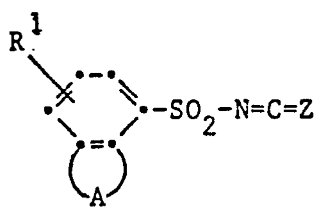

(IV)

worin A, $R^1$ und Z die unter Formel I gegebene Bedeutung haben, in Gegenwart einer Base mit einem Aminopyrimidin oder -triazin der Formel V

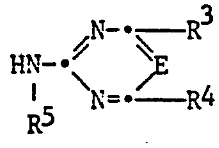

(V)

worin E, $R^2$, $R^3$ und $R^4$ die unter Formel I gegebene Bedeutung haben, umsetzt und gegebenenfalls in die Salze überführt.

16. Verfahren zur Herstellung der Verbindungen der Formel I gemäss Anspruch 1, dadurch gekennzeichnet, dass man ein anelliertes N-Phenylsulfonylcarbamat der Formel VI

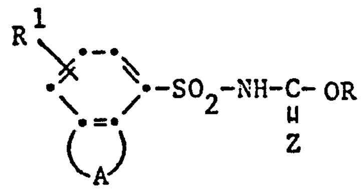

(VI)

worin A, $R_1$ und Z die unter Formel I gegebene Bedeutung haben und R für Phenyl, Alkyl oder substituiertes Phenyl steht, mit einem Aminopyrimidin oder -triazin der oben angegebenen Formel V umsetzt, und gegebenenfalls in die Salze überführt.

17. Verfahren zur Herstellung von Additionssalzen der Formel I gemäss einem der Ansprüche 14 bis 16, dadurch gekennzeichnet, dass man einen Sulfonylharnstoff der Formel I mit einem Amin, einem Alkalimetall- oder Erdalkalimetallhydroxid oder einer quaternären Ammoniumbase umsetzt.

18. Ein herbizides und den Pflanzenwuchs hemmendes Mittel, dadurch gekennzeichnet, dass es neben Träger- und/oder anderen Zuschlagstoffen als Wirkstoff mindestens einen anellierten N-Phenylsulfonyl-N'-pyrimidinyl- oder -triazinyl-harnstoff der Formel I, Anspruch I, enthält.

19. Die Verwendung der Wirkstoffe der Formel I, gemäss Anspruch 1, oder sie enthaltender Mittel zur Bekämpfung unerwünschten Pflanzenwachstums.

20. Die Verwendung der Wirkstoffe der Formel I, gemäss Anspruch 1, oder sie enthaltender Mittel zur Hemmung des Pflanzenwachstums.

21. Die Verwendung der Wirkstoffe der Formel I, gemäss Anspruch 1, oder sie enthaltender Mittel zur Beeinflussung des Pflanzenwachstums zum Zweck der Ertragssteigerung.

22. Die Verwendung der Wirkstoffe der Formel I, oder sie enthaltender Mittel, gemäss Anspruch 19, zur selektiven pre- oder postemergenten Bekämpfung von Unkräutern in Nutzpflanzenkulturen.

23. Anellierte Phenylsulfonylchloride der Formel X

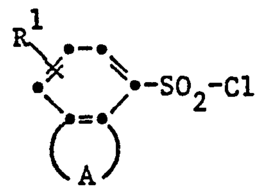

(X),

worin A und $R^1$ die unter Formel I angegebene Bedeutung haben.

**Patentansprüche**

für den Vertragsstaat AT

1. Ein herbizides und den Pflanzenwuchs hemmendes Mittel, dadurch gekennzeichnet, dass es neben Träger- und/oder anderen Zuschlagstoffen als Wirkstoff mindestens einen anellierten N-Phenylsulfonyl-N'-pyrimidinyl- oder -triazinyl-harnstoff der Formel I

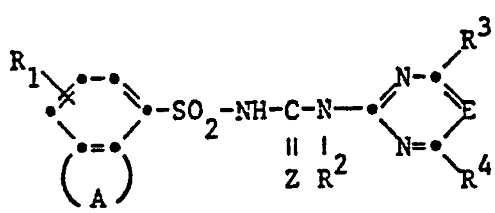

(I),

oder ein Salz davon enthält,
worin
Z Sauerstoff oder Schwefel,
E Stickstoff oder =CH-,
$R^1$ Wasserstoff, Halogen, Nitro, $C_1$-$C_4$-Alkyl $C_1$-$C_4$-Halogenalkyl, Cyan oder eine Gruppe -X-$R^5$, -CO-X-$R^6$,

-CO-NR⁷R⁸, -SO-R⁹ oder -$SO_2$-R¹⁰,

$R^2$ Wasserstoff, $C_1$-$C_4$-Alkyl oder $C_1$-$C_4$-Alkoxy,

$R^3$ $C_1$-$C_4$-Alkyl, $C_1$-$C_4$-Halogenalkyl, $C_1$-$C_4$-Alkoxy, $C_1$-$C_4$-Halogen-alkoxy oder $C_3$-$C_6$-Cycloalkyl,

$R^4$ Wasserstoff, Halogen, $C_1$-$C_4$-Halogenalkyl, $C_1$-$C_4$-Alkoxy, $C_1$-$C_4$-Halogenalkoxy, $C_2$-$C_4$-Alkoxyalkoxy, $C_3$-$C_6$-Cycloalkyl oder -$NR^{11}R^{12}$,

$R^5$ $C_3$-$C_5$-Alkinyl oder unsubstituiertes oder durch Halogen oder $C_1$-$C_4$-Alkoxy substituiertes $C_1$-$C_4$-Alkyl oder $C_3$-$C_5$-Alkenyl,

$R^6$ und $R^9$ $C_1$-$C_4$-Alkyl, $C_1$-$C_4$-Halogenalkyl, $C_2$-$C_4$-Alkoxyalkyl, $C_3$-$C_5$-Alkenyl, $C_3$-$C_5$-Alkinyl, Phenyl oder Benzyl,

$R^7$ und $R^8$ unabhängig voneinander Wasserstoff, $C_1$-$C_4$-Alkyl, $C_1$-$C_4$-Halogenalkyl, $C_2$-$C_4$-Alkoxyalkyl, $C_3$-$C_5$-Alkenyl, $C_3$-$C_5$-Alkinyl, Phenyl oder Benzyl,

$R^{10}$ $C_1$-$C_4$-Alkyl, $C_1$-$C_4$-Alkoxy, $C_1$-$C_4$-Halogenalkoxy oder -$NR^{13}R^{14}$,

$R^{11}$ und $R^{12}$ unabhängig voneinander Wasserstoff oder $C_1$-$C_4$-Alkyl,

$R^{13}$ und $R^{14}$ unabhängig voneinander Wasserstoff, $C_1$-$C_4$-Alkyl, $C_1$-$C_4$-Halogenalkyl, $C_2$-$C_4$ -Alkoxyalkyl, $C_3$-$C_5$-Alkenyl, $C_3$-$C_5$-Alkinyl, Phenyl oder Benzyl,

X Sauerstoff oder Schwefel bedeuten und

A für eine gegebenenfalls substituierte 4-atomige ungesättigte Brücke der Formel -CH=CH-Y- steht, worin Y das zweiatomige Brückenglied -O-$SO_2$- bedeutet und die Substituenten der Brücke A ausgewählt sind aus der Gruppe Halogen, Cyan, $C_1$-$C_4$-Alkyl, $C_1$-$C_4$-Halogenalkyl, $C_1$-$C_4$-Alkoxy $C_1$-$C_4$-Alkyl-carbonyl, $C_1$-$C_4$-Alkoxycarbonyl, $C_1$-$C_4$-Alkylthiocarbonyl, Carbamoyl, $C_1$-$C_4$-Alkylaminocarbonyl, Di-$C_1$-$C_4$-alkylaminocarbonyl, $C_1$-$C_4$-Alkyl-sulfonyl, $C_3$-$C_5$-Alkenyl oder $C_3$-$C_5$-Alkinyl.

2. Mittel gemäss Anspruch 1, dadurch gekennzeichnet, dass die Substituenten der Brücke A Halogen oder $C_1$-$C_4$-Alkyl sind.

3. Mittel gemäss Anspruch 1, dadurch gekennzeichnet, dass Z Sauerstoff bedeutet.

4. Mittel gemäss Anspruch 1, dadurch gekennzeichnet, dass $R^1$ Wasserstoff oder Halogen bedeutet.

5. Mittel gemäss Anspruch 1, dadurch gekennzeichnet, dass $R^2$ für Wasserstoff steht.

6. Mittel gemäss Anspruch 1, dadurch gekennzeichnet, dass $R^3$ und $R^4$ unabhängig voneinander $C_1$-$C_4$-Alkyl, $C_1$-$C_4$-Alkoxy, Di-$C_1$-$C_4$-al-kylamino, $C_1$-$C_4$-Halogenalkoxy oder Halogen bedeuten und zusammen höchstens 4 Kohlenstoffatome enthalten.

7. Mittel gemäss Anspruch 1, dadurch gekennzeichnet, dass Z Sauerstoff, $R^1$ Wasserstoff oder Halogen, $R^2$ Wasserstoff und $R^3$ und $R^4$ unabhängig voneinander $C_1$-$C_4$-Alkyl, $C_1$-$C_4$-Alkoxy, Di-$C_1$-$C_4$-alkylamino, $C_1$-$C_4$-Halogenalkoxy oder Halogen, welche zusammen höchstens 4 Kohlenstoffatome enthalten, bedeuten.

8. Mittel gemäss Anspruch 1, dadurch gekennzeichnet, dass Z Sauerstoff, $R^1$ Wasserstoff, Halogen oder $C_1$-$C_4$-Alkoxycarbonyl, $R^2$ Wasserstoff, $R^3$ und $R^4$ unabhängig voneinander $C_1$-$C_4$-Alkyl, $C_1$-$C_4$-Alkoxy, Di-$C_1$-$C_4$-alkylamino, $C_1$-$C_4$-Halogenalkoxy oder Halogen, welche zusammen höchstens 4 Kohlenstoffatome enthalten, bedeuten und die Brücke A gegebenenfalls durch Halogen oder $C_1$-$C_4$-Alkyl substituiert ist.

9. Mittel gemäss Anspruch 1, dadurch gekennzeichnet, dass es einen Wirkstoff aus der Reihe N-(3-Methyl-2,2-dioxo-1,2-benzoxathiin-8-ylsulfonyl)-N'-(4-methoxy-6-methyl-1,3,5-triazin-2-yl)-harnstoff oder N-(6-Brom-3-methyl-2,2-dioxo-1,2-benzoxathiin-8-ylsulfonyl)-N'-(4-methoxy-6-methyl-1,3,5-triazin-2-yl)-harnstoff enthält.

10. Verfahren zur Herstellung der Verbindungen der Formel I nach Anspruch 1, dadurch gekennzeichnet, dass man entweder

a) ein anelliertes Phenylsulfonanid der Formel II

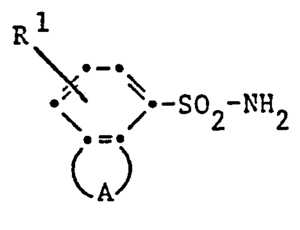

(II)

worin $R^1$ und A die unter Formel I gegebene Bedeutung haben, in Gegenwart einer Base mit einem N-Pyrimidinyl- oder -triazinylcarbamat der Formel III

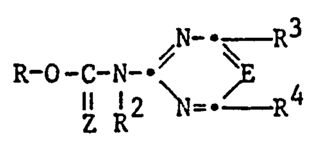

(III)

worin E, $R^2$, $R^3$, $R^4$ und Z die unter Formel I gegebene Bedeutung haben und R für Phenyl, Alkyl oder substituiertes Phenyl steht, umsetzt oder

b) ein anelliertes Phenylsulfonylisocyanat oder -isothiocyanat der Formel IV

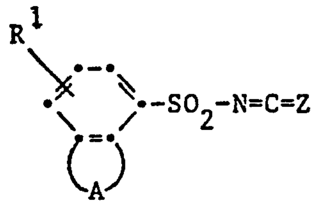

(IV)

worin A, $R^1$ und Z die unter Formel I gegebene Bedeutung haben, in Gegenwart einer Base mit einem Aminopyrimidin oder -triazin der Formel V

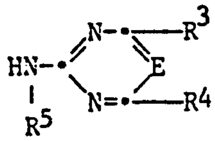

(V)

worin E, $R^2$, $R^3$ und $R^4$ die unter Formel I gegebene Bedeutung haben, umsetzt oder
c) ein anelliertes N-Phenylsulfonylcarbamat der Formel VI

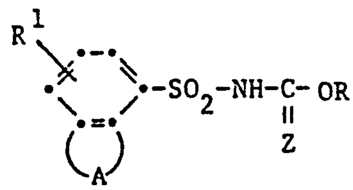

(VI)

worin A, $R^1$ und Z die unter Formel I gegebene Bedeutung haben und R für Phenyl, Alkyl oder substituiertes Phenyl steht, mit einem Aminopyrimidin oder -triazin der oben angegebenen Formel V umsetzt, und gegebenenfalls in die Salze überführt indem man einen Sulfonylharnstoff der Formel I mit einem Amin, einem Alkalimetall- oder Erdalkalimetallhydroxid oder einer quaternären Ammoniumbase umsetzt.

11. Die Verwendung der Wirkstoffe der Formel I nach Anpsruch 1, oder sie enthaltender Mittel zur Bekämpfung unerwünschten Pflanzenwachstums.

12. Die Verwendung der Wirkstoffe der Formel I nach Anspruch 1, oder sie enthaltender Mittel zur Hemmung des Pflanzenwachstums.

13. Die Verwendung der Wirkstoffe der Formel I nach Anspruch 1, oder sie enthaltender Mittel zur Beeinflussung des Pflanzenwachstums zum Zweck der Ertragssteigerung.

14. Die Verwendung gemäss Anspruch 11 zur selektiven pre- oder postemergenten Bekämpfung von Unkräutern in Nutzpflanzenkulturen.

**Claims**

for the Contracting States: BE, CH, LI, DE, FR, GB, IT, NL, SE

1. A fused N-phenylsulfonyl-N'-pyrimidinylurea or N-phenylsulfonyl-N'-triazinylurea of the formula I

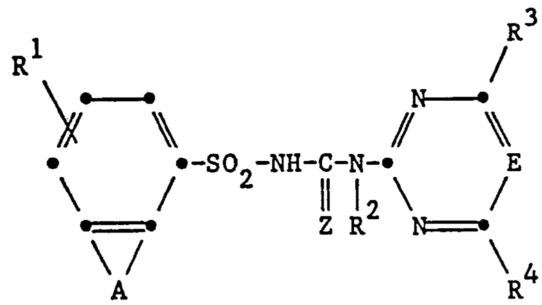

(I),

wherein

Z is oxygen or sulfur,

E is nitrogen or =CH-,

$R^1$ is hydrogen, halogen, nitro, $C_1$-$C_4$ alkyl, $C_1$-$C_4$ haloalkyl, cyano, or a $-X-R^5$, $-CO-X-R^6$, $-CO-NR^7R^8$, $-SO-R^9$ or $-SO_2-R^{10}$ group,

$R^2$ is hydrogen, $C_1$-$C_4$ alkyl or $C_1$-$C_4$ alkoxy,

$R^3$ is $C_1$-$C_4$ alkyl, $C_1$-$C_4$ haloalkyl, $C_1$-$C_4$ alkoxy, $C_1$-$C_4$ haloalkoxy or $C_3$-$C_6$ cycloalkyl,

$R^4$ is hydrogen, halogen, $C_1$-$C_4$ haloalkyl, $C_1$-$C_4$ alkoxy, $C_1$-$C_4$ haloalkoxy, $C_2$-$C_4$ alkoxyalkoxy, $C_3$-$C_6$ cycloalkyl or $-NR^{11}R^{12}$,

$R^5$ is $C_3$-$C_5$ alkynyl or $C_1$-$C_4$ alkyl which is unsubstituted or substituted by halogen or $C_1$-$C_4$ alkoxy, or is $C_3$-$C_5$ alkenyl, which is unsubstituted or is substituted by halogen or $C_1$-$C_4$ alkoxy,

$R^6$ and $R^9$ are each independently $C_1$-$C_4$ alkyl, $C_1$-$C_4$ haloalkyl $C_2$-$C_4$ alkoxyalkyl, $C_3$-$C_5$ alkenyl, $C_3$-$C_5$ alkynyl, phenyl or benzyl,

$R^7$ and $R^8$ are each independently hydrogen, $C_1$-$C_4$ alkyl, $C_1$-$C_4$ haloalkyl, $C_2$-$C_4$ alkoxyalkyl, $C_3$-$C_5$ alkenyl, $C_3$-$C_5$ alkynyl, phenyl or benzyl,

$R^{10}$ is $C_1$-$C_4$ alkyl, $C_1$-$C_4$ alkoxy, $C_1$-$C_4$ haloalkoxy or $-NR^{13}R^{14}$,

$R^{11}$ and $R^{12}$ are each independently hydrogen or $C_1$-$C_4$ alkyl,

$R^{13}$ and $R^{14}$ are each independently hydrogen $C_1$-$C_4$ alkyl, $C_1$-$C_4$ haloalkyl, $C_2$-$C_4$ alkoxyalkyl, $C_3$-$C_5$ alkenyl, $C_3$-$C_5$ alkynyl, phenyl or benzyl,

X is oxygen or sulfur, and

A is an unsubstituted or substituted unsaturated bridge of 4 atoms of the formula -CH=CH-Y-, wherein Y is a bridge member of 2 atoms $-O-SO_2-$, and the substituents of the bridge A are selected from the group consisting of halogen, cyano, $C_1$-$C_4$ alkyl, $C_1$-$C_4$ haloalkyl, $C_1$-$C_4$ alkoxy, $C_1$-$C_4$ alkylcarbonyl, $C_1$-$C_4$ alkoxycarbonyl, $C_1$-$C_4$ alkylthiocarbonyl, carbamoyl, $C_1$-$C_4$ alkylaminocarbonyl, di($C_1$-$C_4$)alkylaminocarbonyl, $C_1$-$C_4$ alkylsulfonyl, $C_3$-$C_5$ alkenyl or $C_3$-$C_5$ alkynyl; or a salt thereof.

2. A compound according to claim 1, wherein the substituents of the bridge A are halogen or $C_1$-$C_4$ alkyl.

3. A compound according to claim 1, wherein Z is oxygen.

4. A compound according to claim 1, wherein $R^1$ is hydrogen or halogen.

5. A compound according to claim 1, wherein $R^2$ is hydrogen.

6. A compound according to claim 1, wherein each of $R^3$ and $R^4$ independently of the other is $C_1$-$C_4$ alkyl, $C_1$-$C_4$ alkoxy, di($C_1$-$C_4$)-alkylamino, $C_1$-$C_4$ haloalkoxy or halogen, and together contain not more than 4 carbon atoms.

7. A compound according to claim 1, wherein Z is oxygen, $R^1$ is hydrogen or halogen, $R^2$ is hydrogen, each of $R^3$ and $R^4$ independently of the other is $C_1$-$C_4$ alkyl, $C_1$-$C_4$ alkoxy, di($C_1$-$C_4$) alkylamino, $C_1$-$C_4$ haloalkoxy or halogen, and together contain not more than 4 carbon atoms.

8. A compound according to claim 1 wherein Z is oxygen, $R^1$ is hydrogen, $C_1$-$C_4$-alkoxycarbonyl or halogen, $R^2$ is hydrogen, each of $R^3$ and $R^4$ independently of the other is $C_1$-$C_4$ alkyl, $C_1$-$C_4$ alkoxy, di($C_1$-$C_4$) alkylamino, $C_1$-$C_4$ haloalkoxy or halogen, and together contain not more than 4 carbon atoms, and the bridge A is unsubstituted or substituted by halogen or $C_1$-$C_4$ alkyl.

9. N-(3-Methyl-2,2-dioxo-1,2-benzoxathiin-8-ylsulfonyl)-N'-(4-methoxy-6-methyl-1,3,5-triazin-2-yl)urea according to claim 1.

10. N-(6-Bromo-3-methyl-2,2-dioxo-1,2-benzoxathiin-8-ylsulfonyl)-N'-(4-methoxy-6-methyl-1,3,5-triazin-2-yl)urea according to claim 1.

11. A fused phenylsulfonamide of the general formula II

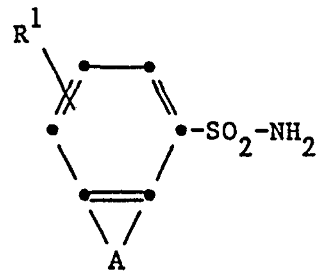

(II),

wherein $R^1$ and A are as defined for formula I in claim 1.

12. A fused phenylsulfonyl isocyanate or phenylsulfonyl isothiocyanate of the general formula IV

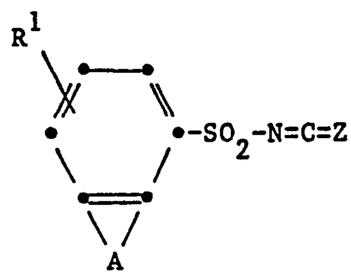

(IV),

wherein A, $R^1$ and Z are as defined for formula I in claim 1.

13. A fused phenylsulfonylcarbamate of the general formula VI

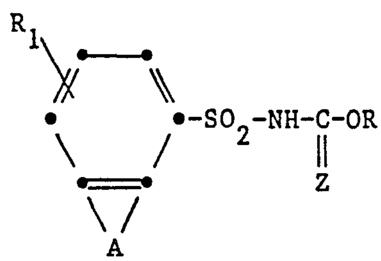

(VI),

wherein A, $R^1$ and Z are as defined for formula I in claim 1, and R is phenyl, alkyl or substituted phenyl.

14. A process for the preparation of a compound of formula I according to claim 1, which comprises reacting a fused phenylsulfonamide of the formula II

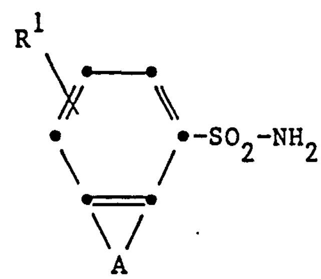

(II),

wherein $R^1$ and A are as defined for formula I, with an N-pyrimidinylcarbamate or N-triazinylcarbamate of the formula III

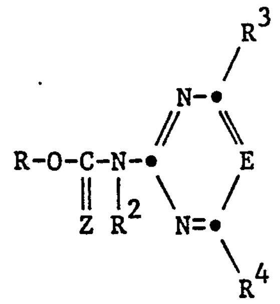

(III),

wherein E, $R^2$, $R^3$, $R^4$ and Z are as defined for formula I and R is phenyl, alkyl or substituted phenyl, in the presence of a base, and, if desired, converting the compound of formula I so obtained into a salt thereof.

15. A process for the preparation of a compound of the formula I according to claim 1, which comprises reacting a fused phenylsulfonyl isocyanate or phenylsulfonyl isothiocyanate of the formula IV

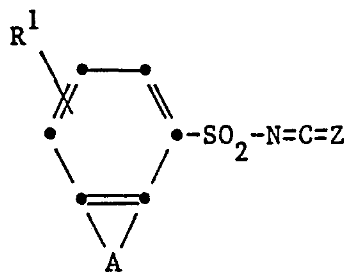

(IV),

wherein A, $R^1$ and Z are as defined for formula I, with an aminopyridine or aminotriazine or the formula V

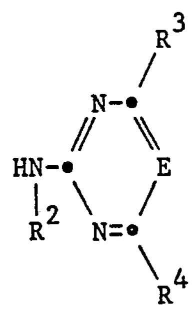

(V)

wherein E, $R^2$, $R^3$ and $R^4$ are as defined for formula I, optionally in the presence of a base, and, if desired, converting the compound of formula I so obtained into a salt thereof.

16. A process for the preparation of a compound of formula I according to claim 1, which comprises reacting a fused N-phenylsulfonylcarbamate of the formula VI

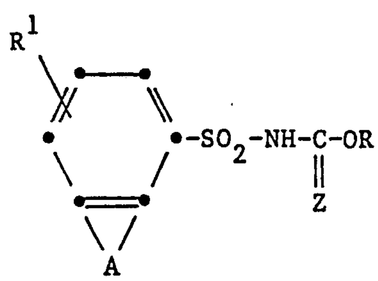

(VI),

wherein A, $R^1$ and Z are as defined for formula I and R is phenyl, alkyl or substituted phenyl, with an aminopyrimidine or aminotriazine of the formula V, and, if desired, converting the compound of formula I so obtained into a salt thereof.

17. A process for the preparation of an addition salt of the formula I according to any one of claims 14 to 16,

which comprises reacting a sulfonylurea of the formula I with an amide, an alkali metal hydroxide or alkaline earth metal hydroxide or with a quaternary ammonium base.

18. A herbicidal and plant growth inhibiting composition which contains at least one fused N-phenylsulfonyl-N'-pyrimidinylurea or N-phenylsulfonyl-N'-triazinylurea of the formula I as claimed in claim 1, together with carriers and/or other adjuvants.

19. The use of a compound of the formula I according to claim 1, or of a composition containing such a compound, for controlling undesirable plant growth.

20. The use of a compound of the formula I according to claim 1, or of a composition containing such a compound, for inhibiting plant growth.

21. The use of a compound of the formula I according to claim 1, or of a composition containing such a compound, for influencing plant growth to increase yield.

22. The use of a compound of the formula I, or of a composition containing such a compound, for selectively controlling weeds pre- or postemergence in crops of useful plants.

23. A fused phenylsulfonyl chloride of the formula X

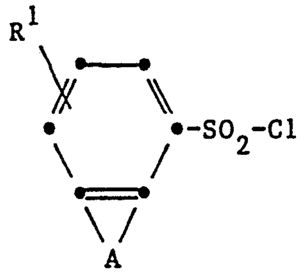

(X) ,

wherein A and $R^1$ are as defined for formula I.

**Claims**

for the Contracting State: AT

1. A herbicidal and plant growth inhibiting composition which contains, as active ingredient, at least one fused N-phenylsulfonyl-N'-pyrimidinylurea or N-phenylsulfonyl-N'-triazinylurea of the formula I

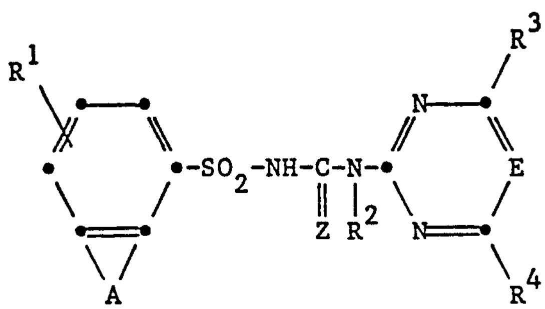

(I),

wherein

Z is oxygen or sulfur,

E is nitrogen or =CH-,

$R^1$ is hydrogen, halogen, nitro, $C_1$-$C_4$ alkyl, $C_1$-$C_4$ haloalkyl, cyano, or a -X-$R^5$, -CO-X-$R^6$, -CO-$NR^7R^8$, -SO-$R^9$ or -$SO_2$-$R^{10}$ group

$R^2$ is hydrogen, $C_1$-$C_4$ alkyl or $C_1$-$C_4$ alkoxy,

$R^3$ is $C_1$-$C_4$ alkyl, $C_1$-$C_4$ haloalkyl, $C_1$-$C_4$ alkoxy, $C_1$-$C_4$ haloalkoxy or $C_3$-$C_6$ cycloalkyl,

$R^4$ is hydrogen, halogen, $C_1$-$C_4$ haloalkyl, $C_1$-$C_4$ alkoxy, $C_1$-$C_4$ haloalkoxy, $C_2$-$C_4$ alkoxyalkoxy, $C_3$-$C_6$ cycloalkyl or -$NR^{11}R^{12}$,

$R^5$ is $C_3$-$C_5$ alkynyl or $C_1$-$C_4$ alkyl which is unsubstituted or substituted by halogen or $C_1$-$C_4$-alkoxy, or is $C_3$-$C_5$ alkenyl, which is unsubstituted or is substituted by halogen or $C_1$-$C_4$-alkoxy,

$R^6$ and $R^9$ are each independently $C_1$-$C_4$ alkyl, $C_1$-$C_4$ haloalkyl, $C_2$-$C_4$ alkoxyalkyl, $C_3$-$C_5$ alkenyl, $C_3$-$C_5$ alkynyl, phenyl or benzyl,

$R^7$ and $R^8$ are each independently hydrogen, $C_1$-$C_4$ alkyl, $C_1$-$C_4$ haloalkyl, $C_2$-$C_4$ alkoxyalkyl, $C_3$-$C_5$ alkenyl, $C_3$-

$C_5$ alkynyl, phenyl or benzyl,

$R^{10}$ is $C_1$-$C_4$ alkyl, $C_1$-$C_4$ alkoxy, $C_1$-$C_4$ haloalkoxy or $-NR^{13}R^{14}$,

$R^{11}$ and $R^{12}$ are each independently hydrogen or $C_1$-$C_4$ alkyl,

$R^{13}$ and $R^{14}$ are each independently hydrogen, $C_1$-$C_4$ alkyl, $C_1$-$C_4$ haloalkyl, $C_2$-$C_4$ alkoxyalkyl, $C_3$-$C_5$ alkenyl, $C_3$-$C_5$ alkynyl, phenyl or benzyl,

X is oxygen or sulfur, and

A is an unsubstituted or substituted unsaturated bridge of 4 atoms of the formula -CH=CH-Y-, wherein Y is the bridge member of 2 atoms, $-O-SO_2-$, and the substituents of the bridge A are selected from the group consisting of halogen, cyano, $C_1$-$C_4$ alkyl, $C_1$-$C_4$ haloalkyl, $C_1$-$C_4$ alkoxy, $C_1$-$C_4$ alkylcarbonyl, $C_1$-$C_4$ alkoxycarbonyl, $C_1$-$C_4$ alkylthiocarbonyl, carbamoyl, $C_1$-$C_4$ alkylaminocarbonyl, di($C_1$-$C_4$)alkylaminocarbonyl, $C_1$-$C_4$ alkylsulfonyl, $C_3$-$C_5$ alkenyl or $C_3$-$C_5$ alkynyl; or a salt thereof together with carriers and/or other adjuvants.

2. A composition according to claim 1, wherein the subititutents of the bridge A are halogen or $C_1$-$C_4$ alkyl.

3. A composition according to claim 1, wherein Z is oxygen.

4. A composition according to claim 1, wherein $R^1$ is hydrogen or halogen.

5. A composition according to claim 1, wherein $R^2$ is hydrogen.

6. A composition according to claim 1, wherein each of $R^3$ and $R^4$ independently of the other is $C_1$-$C_4$ alkyl, $C_1$-$C_4$ alkoxy, di($C_1$-$C_4$) alkylamino, $C_1$-$C_4$ haloalkoxy or halogen, and together contain not more than 4 carbon atoms.

7. A composition according to claim 1, wherein Z is oxygen, $R^1$ is hydrogen or halogen, $R^2$ is hydrogen, each of $R^3$ and $R^4$ independently of the other is $C_1$-$C_4$ alkyl, $C_1$-$C_4$ alkoxy, di($C_1$-$C_4$) alkylamino, $C_1$-$C_4$ haloalkoxy or halogen, and together contain not more than 4 carbon atoms.

8. A composition according to claim 1 wherein Z is oxygen, $R^1$ is hydrogen, $C_1$-$C_4$ alkoxycarbonyl or halogen $R^2$ is hydrogen, each of $R^3$ and $R^4$ independently of the other is $C_1$-$C_4$ alkyl, $C_1$-$C_4$ alkoxy, di($C_1$-$C_4$) alkylamino, $C_1$-$C_4$ haloalkoxy or halogen, and together contain not more than 4 carbon atoms, and the bridge A is unsubstituted or substituted by halogen or $C_1$-$C_4$ alkyl.

9. A composition according to claim 1, which contains a compound selected from the group consisting of N-(3-methyl-2,2-dioxo-1,2-benzoxathiin-8-ylsulfonyl)-N'-(4-methoxy-6-methyl-1,3,5-triazin-2-yl)urea and N-(6-bromo-3-methyl-2,2-dioxo-1,2-benzoxathiin-8-ylsulfonyl)-N'-(4-methoxy-6-methyl-1,3,5-triazin-2-yl)urea.

10. A process for the preparation of a compound of formula I according to claim 1, which comprises either reacting

a) a fused phenylsulfonamide of the formula II

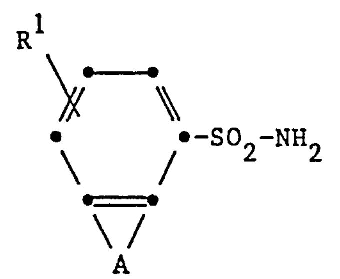

(II),

wherein $R^1$ and A are as defined for formula I, with an N-pyrimidinylcarbamate or N-triazinylcarbamate of the formula III

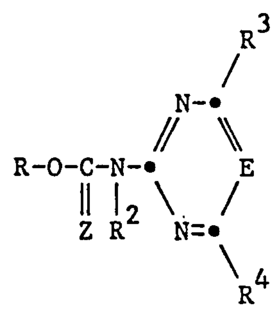

(III),

wherein E, $R^2$, $R^3$, $R^4$, and Z are as defined for formula I and R is phenyl, alkyl or substituted phenyl, in the presence of a base, or

b) reacting a fused phenylsulfonyl isocyanate or phenylsulfonyl isothiocyanate of the formula IV

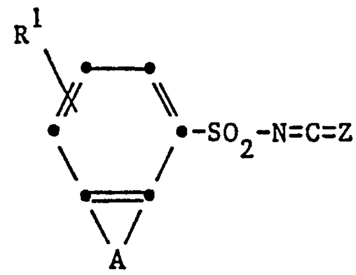

(IV),

wherein A, $R^1$ and Z are as defined for formula I, with an aminopyridine or aminotriazine or the formula V

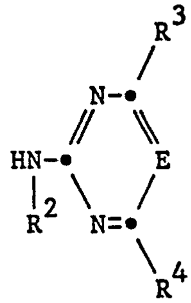

(V)

wherein E, $R^2$, $R^3$ and $R^4$ are as defined for formula I, or
c) reacting a fused N-phenylsulfonylcarbamate of the formula VI

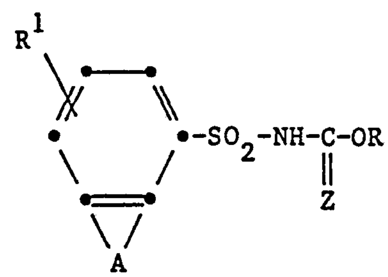

(VI),

wherein A, $R^1$ and Z are as defined for formula I and R is phenyl, alkyl or substituted phenyl, with an aminopyrimidine or aminotriazine of the formula V above and, if desired, converting the compound of formula I so obtained into a salt thereof by reacting a sulfonylurea of the formula I with an amide, an alkali metal hydroxide or alkaline earth metal hydroxide or with a quaternary ammonium base.

11. The use of a compound of the formula I according to claim 1, or of a composition containing such a compound, for controlling undesirable plant growth.

12. The use of a compound of the formula I according to claim 1, or of a composition containing such a compound, for inhibiting plant growth.

13. The use of a compound of the formula I according to claim 1, or of a composition containing such a compound, for influencing plant growth to increase yield.

14. The use of a compound of the formula I, or of a composition containing such a compound, for selectively controlling weeds pre- or postemergence in crops of useful plant.

**Revendications**

pour les Etats Contractants BE, CH, LI, DE, FR, GB, IT, NL, SE

1. N-phénylsulfonyl-N'-pyrimidinyl- et -triazinylurées condensées de formule I

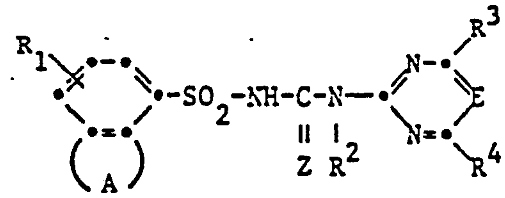 (I),

dans laquelle

Z est l'oxygène ou le soufre,

E est l'azote ou =CH-,

$R^1$ est un hydrogène, halogène, nitro, alkyle $C_1$-$C_4$, halogénoalkyle $C_1$-$C_4$, cyano ou un groupe -X-$R^5$, -CO-X-$R^6$, -CO-$NR^7R^8$, -SO-$R^9$ ou -$SO_2$-$R^{10}$,

$R^2$ est un hydrogène alkyle $C_1$-$C_4$ ou alcoxy $C_1$-$C_4$,

$R^3$ est un alkyle $C_1$-$C_4$, halogénoalkyle $C_1$-$C_4$, alcoxy $C_1$-$C_4$ ou cycloalkyle $C_3$-$C_6$,

$R^4$ est un hydrogène, halogène, halogénoalkyle $C_1$-$C_4$, alcoxy $C_1$-$C_4$, halogénoalcoxy $C_1$-$C_4$, alcoxyalcoxy $C_2$-$C_4$, cycloalkyle $C_3$-$C_6$ ou -$NR^{11}R^{12}$,

$R^5$ est un alcynyle $C_3$-$C_5$ ou un alcényle $C_3$-$C_5$ ou un alkyle $C_1$-$C_4$ non substitués ou substitués par un halogène ou un alcoxy $C_1$-$C_4$,

$R^6$ et $R^9$ sont un alkyle $C_1$-$C_4$, halogénoalkyle $C_1$-$C_4$, alcoxyalkyle $C_2$-$C_4$, alcényle $C_3$-$C_5$, alcynyle $C_3$-$C_5$, phényle ou benzyle,

$R^7$ et $R^8$ indépendamment l'un de l'autre, sont un hydrogène, alkyle $C_1$-$C_4$, halogénoalkyle $C_1$-$C_4$, alcoxyalkyle $C_2$-$C_4$, alcényle $C_3$-$C_5$, alcynyle $C_3$-$C_5$, phényle ou benzyle,

$R^{10}$ est un alkyle $C_1$-$C_4$, alcoxy $C_1$-$C_4$, halogénoalcoxy $C_1$-$C_4$ ou -$NR^{13}R^{14}$,

$R^{11}$ et $R^{12}$ sont, independamment l'une de l'autre, un hydrogène ou alkyle $C_1$-$C_4$,

$R^{13}$ et $R^{14}$ sont indépendamment l'un de l'autre, un alkyle $C_1$-$C_4$, halogénoalkyle $C_1$-$C_4$, alcoxyalkyle $C_2$-$C_4$, alcényle $C_3$-$C_5$, alcynyle $C_3$-$C_5$, phényle ou benzyle

X est l'oxygène ou le soufre et

A est un pont insaturé à 4 atomes, le cas échéant substitué de formule -CH=CH-Y-, dans laquelle Y est le membre de pont diatomique -O-$SO_2$- et les substituants du pont A sont choisis dans le groupe des halogène, cyano, alkyle $C_1$-$C_4$, halogénoalkyle $C_1$-$C_4$, alcoxy $C_1$-$C_4$, alkylcarbonyle $C_1$-$C_4$, alcoxycarbonyle $C_1$-$C_4$, alkylthiocarbonyle $C_1$-$C_4$, carbamoyle, alkylaminocarbonyle $C_1$-$C_4$, dialkylaminocarbonyle $C_1$-$C_4$, alkylsulfonyle $C_1$-$C_4$, alcényle $C_3$-$C_5$ ou alcynyle $C_3$-$C_5$; ainsi que les sels de ces composés.

2. Composés selon la revendication 1, caractérisés en ce que les substituants du pont A sont un halogène ou un alkyle $C_1$-$C_4$.

3. Composés selon la revendication 1, caractérisés en ce que Z représente l'oxygène.

4. Composés selon la revendication 1, caractérisés en ce que $R^1$ représente l'hydrogène ou un halogène.

5. Composés selon la revendication 1, caractérisés en ce que $R^2$ représente l'hydrogène.

6. Composés selon la revendication 1, caractérisés en ce que $R^3$ et $R^4$, indépendamment l'un de l'autre, représentent un alkyle $C_1$-$C_4$, alcoxy $C_1$-$C_4$, di-alkyl-$C_1$-$C_4$-amino, halogénoalcoxy $C_1$-$C_4$ ou halogène et contiennent ensemble 4 atomes de carbone au plus.

7. Composés selon la revendication 1, caractérisés en ce que Z est l'oxygène, $R^1$ l'hydrogène ou un halogène, $R^2$ l'hydrogène et $R^3$ et $R^4$, indépendamment l'un de l'autre, représentent un alkyle $C_1$-$C_4$, alcoxy $C_1$-$C_4$, di-alkyl-$C_1$-$C_4$-amino, halogénoalcoxy $C_1$-$C_4$ ou halogène, et contiennent ensemble 4 atomes de carbone au plus.

8. Composés selon la revendication 1, caractérisés en ce que Z est l'oxygène, $R^1$ l'hydrogène, un halogène ou alcoxycarbonyle $C_1$-$C_4$, $R^2$ l'hydrogène, $R^3$ et $R^4$, indépendamment l'un de l'autre, un alkyle $C_1$-$C_4$, alcoxy $C_1$-$C_4$, di-alkyl-$C_1$-$C_4$-amino, halogénoalcoxy $C_1$-$C_4$ ou halogène, qui contiennent ensemble 4 atomes de carbone au plus et le pont A est substitué, le cas échéant par un halogène ou un alkyle $C_1$-$C_4$.

9. N-(3-méthyl-2,2-dioxo-1,2-benzoxathiine-8-ylsulfonyl)-N'-(4-méthoxy-6-méthyl-1,3,5-triazine-2-yl)-urée selon la revendication 1.

10. N-(6-bromo-3-méthyl-2,2-dioxo-1,2-benzoxa-thiine-8-ylsulfonyl)-N'-(4-méthoxy-6-méthyl-1,3,5-tria-zine-2-yl)-urée selon la revendication 1.

11. Phénylsulfonamides condensés de formule générale II

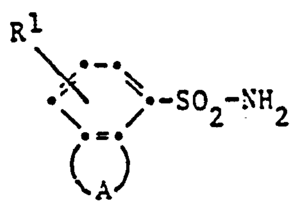

(II),

dans laquelle $R^1$ et A ont la signification donnée dans la revendication 1 pour la formule I.

12. Phénylsulfonylisocyanates et isothiocyanates de formule générale IV

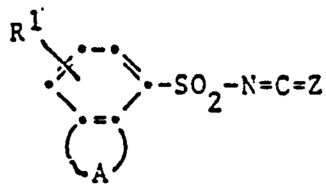

(IV),

dans laquelle A, $R^1$ et Z ont la signification donnée dans la revendication 1 pour la formule I.

13. Phénylsulfonylcarbamates de formule générale VI

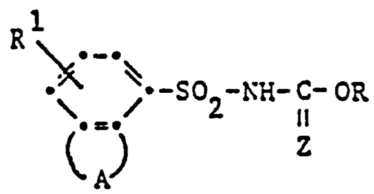

(VI),

dans laquelle A, $R^1$ et Z ont la signification donnée dans la revendication 1 pour la formule I et R est un phényle, alkyle ou phényle substitué.

14. Procédé de préparation de composés de formule I, selon la revendication 1, caractérisé en ce qu'on fait réagir un phénylsulfonamide condensé de formule II

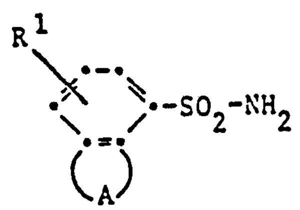

(II),

dans laquelle $R^1$ et A ont la signification indiquée pour la formule I, en présence d'une base avec un N-pyrimidinyl- ou -triazinylcarbamate de formule III

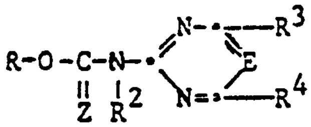

(III),

dans laquelle E, $R^2$, $R^3$, $R^4$ et Z ont la signification donnée pour la formule I et R est un phényle, alkyle ou phényle substitué, et qu'on les transforme, le cas échéant en sels.

15. Procédé de préparation de composés de formule I, selon la revendication 1, caractérisé en ce qu'on fait réagir un phénylsulfonylisocyanate ou isothiocyanate condensé de formule IV

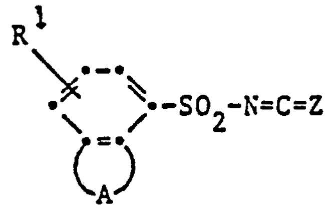

(IV)

dans lequel A, $R^1$ et Z ont la signification indiquée pour la formule I, en présence d'une base avec une aminopyrimidine ou -triazine de formule V

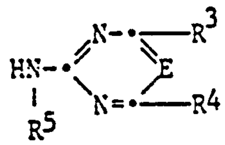

(V),

dans laquelle E, $R^2$, $R^3$ et $R^4$ ont la signification donnée pour la formule I et qu'on les transforme le cas échéant, en sels.

16. Procédé de préparation de composés de formule I, selon la revendication 1, caractérisé en ce qu'on fait réagir un N-phénylsulfonylcarbamate de formule VI

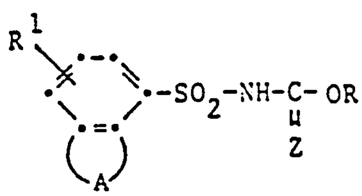

(VI),

dans laquelle A, $R^1$ et Z ont la signification donnée pour la formule I et R est un phényle, alkyle ou phényle substitué, avec une aminopyrimidine ou -triazine de formule V donnée ci-dessus, et qu'on les transforme, le cas échéant, en sels.

17. Procéde de préparation de sels d'addition de formule I selon l'une des revendications 14 à 16, caractérisé en ce qu'on fait réagir une sulfonylurée de formule I avec une amine, un hydroxyde de métal alcalin ou alcalino-terreux ou une base d'ammonium quaternaire.

18. Moyen herbicide et bloquant la croissance des plantes, caractérisé en ce qu'à côté de véhicules et/ou d'autres additifs, il contient comme produit actif au moins une N-phénylsulfonyl-N'-pyrimidinyl- ou triazinylurée de formule I, revendication 1.

19. Utilisation de principes actifs de formule I, selon la revendication 1, ou les moyens qui en contiennent pour combattre la croissance indésirable de plantes.

20. Utilisation de principes actifs de formule I, selon la revendication 1, ou de moyens les contenant pour bloquer la croissance de plantes.

21. Utilisation de principes actifs de formule I, selon la revendication 1, ou de moyens les contenant pour influencer la croissance de plantes dans le but d'augmenter le rendement.

22. Utilisation de principes actifs de formule I, ou de moyens les contenant, selon la revendication 19, pour combattre sélectivement avant ou après la levée des mauvaises herbes dans les cultures de plantes vivrières.

23. Chlorures de phénylsulfonyle de formule X

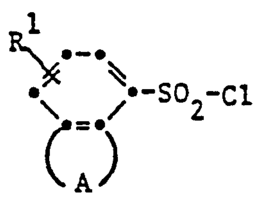

(X),

dans laquelle A et $R^1$ ont la signification donnée pour la formule I.

**Revendications**

pour l'Etat Contractant AT

1. Herbicide et moyen bloquant la croissance de plantes, caractérisé en ce qu'à côté de véhicules et/ou d'autres additifs, il contient comme produit actif au moins une N-phénylsulfonyl-N'-pyrimidinyl- ou triazinylurée de formule I

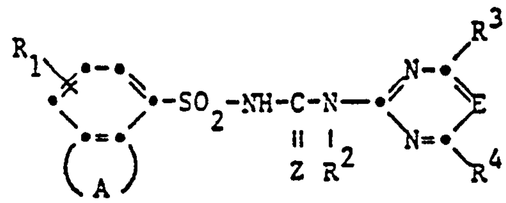

(I),

ou un de ses sels,
dans laquelle
Z est l'oxygène ou le soufre,
E est l'azote ou =CH-,
$R^1$ est un hydrogène, halogène, nitro, alkyle $C_1$-$C_4$, halogénoalkyle $C_1$-$C_4$, cyano ou un groupe -X-$R^5$, -CO-X-$R^6$, -CO-$NR^7R^8$, -SO-$R^9$ ou -$SO_2$-$R^{10}$
$R^2$ est un hydrogène, alkyle $C_1$-$C_4$ ou alcoxy $C_1$-$C_4$,
$R^3$ est un alkyle $C_1$-$C_4$ halogénoalkyle $C_1$-$C_4$, alcoxy $C_1$-$C_4$ halogénoalcoxy $C_1$-$C_4$ ou cycloalkyle $C_3$-$C_6$,
$R^4$ est un hydrogène, halogène, halogénoalkyle $C_1$-$C_4$, alcoxy $C_1$-$C_4$, halogénoalcoxy $C_1$-$C_4$, alcoxyalcoxy $C_2$-$C_4$, cycloalkyle $C_3$-$C_6$ ou -$NR^{11}R^{12}$
$R^5$ est un alcynyle $C_3$-$C_5$ ou un alcényle $C_3$-$C_5$ ou un alkyle $C_1$-$C_4$ non substitués ou substitués par un halogène ou un alcoxy $C_1$-$C_4$,
$R^6$ et $R^9$ sont un alkyle $C_1$-$C_4$, halogénoalkyle $C_1$-$C_4$, alcoxyalkyle $C_2$-$C_4$, alcényle $C_3$-$C_5$, alcynyle $C_3$-$C_5$, phényle ou benzyle,
$R^7$ et $R^8$, indépendamment l'un de l'autre, sont un hydrogène, alkyle $C_1$-$C_4$, halogénoalkyle $C_1$-$C_4$, alcoxyalkyle $C_2$-$C_4$, alcényle $C_3$-$C_5$, alcynyle $C_3$-$C_5$, phényle ou benzyle,
$R^{10}$ est un alkyle $C_1$-$C_4$, alcoxy $C_1$-$C_4$, halogénoalcoxy $C_1$-$C_4$ ou -$NR^{13}R^{14}$,
$R^{11}$ et $R^{12}$ sont, indépendamment l'un de l'autre, un hydrogène ou alkyle $C_1$-$C_4$,
$R^{13}$ et $R^{14}$ sont, indépendamment l'un de l'autre, un alkyle $C_1$-$C_4$, halogénoalkyle $C_1$-$C_4$, alcoxyalkyle $C_2$-$C_4$, alcényle $C_3$-$C_5$, alcynyle $C_3$-$C_5$, phényle ou benzyle
X est l'oxygène ou le soufre, et
A est un pont insaturé à 4 atomes, le cas échéant substitué de formule -CH=CH-Y-, dans laquelle Y est le membre de pont diatomique -O-$SO_2$- et les substituants du pont A sont choisis dans le groupe des halogène, cyano, alkyle $C_1$-$C_4$, halogénoalkyle $C_1$-$C_4$, alcoxy $C_1$-$C_4$, alkylcarbonyle $C_1$-$C_4$, alcoxycarbonyle $C_1$-$C_4$, alkylthiocarbonyle $C_1$-$C_4$, carbamoyle, alkylaminocarbonyle $C_1$-$C_4$, dialkylaminocarbonyle $C_1$-$C_4$, alkylsulfonyle $C_1$-$C_4$, alcényle $C_3$-$C_5$ ou alcynyle $C_3$-$C_5$.

2. Moyen selon la revendication 1, caractérisé en ce que les substituants du pont A sont un halogène ou un alkyle $C_1$-$C_4$.

3. Moyen selon la revendication 1, caractérisé en ce que Z représente l'oxygène.

4. Moyen selon la revendication 1, caractérisé en ce que $R^1$ représente l'hydrogène ou un halogène.

5. Moyen selon la revendication 1, caractérisé en ce que $R^2$ représente l'hydrogène.

6. Moyen selon la revendication 1, caractérisé en ce que $R^3$ et $R^4$, indépendamment l'un de l'autre, representent un alkyle $C_1$-$C_4$, alcoxy $C_1$-$C_4$, di-alkyl-$C_1$-$C_4$-amino, halogénoalcoxy $C_1$-$C_4$ ou halogène et contiennent ensemble 4 atomes de carbone au plus.

7. Moyen selon la revendication 1, caractérisé en ce que Z est l'oxygène, $R^1$ l'hydrogène ou un halogène, $R^2$ l'hydrogène et $R^3$ et $R^4$, indépendamment l'un de l'autre, représentent un alkyle $C_1$-$C_4$, alcoxy $C_1$-$C_4$, di-alkyl-$C_1$-$C_4$-amino, halogénoalcoxy $C_1$-$C_4$ ou halogène, et contiennent ensemble 4 atomes de carbone au plus.

8. Moyen selon la revendication 1, caractérisé en ce que Z est l'oxygène, $R^1$ l'hydrogène, un halogène ou alcoxycarbonyle $C_1$-$C_4$, $R^2$ l'hydrogène, $R^3$ et $R^4$ indépendamment l'un de l'autre, un alkyle $C_1$-$C_4$, alcoxy $C_1$-$C_4$, di-alkyl-$C_1$-$C_4$-amino, halogénoalcoxy $C_1$-$C_4$ ou halogène, qui contiennent ensemble 4 atomes de carbone au plus et le pont A est substitué le cas échéant par un halogène ou un alkyle $C_1$-$C_4$.

9. Moyen selon la revendication 1, caractérisé en ce qu'il contient un principe actif de la série N-(3-méthyl-2,2-dioxo-1,2-benzoxathiine-8-ylsulfonyl)-N'-(4-méthoxy-6-méthyl-1,3,5-triazine-2-yl)-urée ou N-(6-bromo-3-méthyl-2,2-dioxo-1,2-benzoxathiine-8-yl-sulfonyl)-N'-(4-méthoxy-6-méthyl-1,3,5-triazine-2-yl)-urée.

10. Procédé de préparation de composés de formule I selon la revendication 1, caractérisé en ce que, soit
a) on fait réagir un phénylsulfonamide condensé de formule II

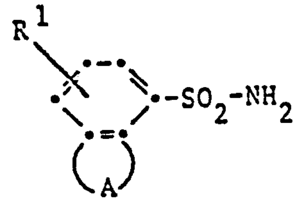

(II),

dans laquelle $R^1$ et A ont la signification indiquée pour la formule I, en présence d'une base avec un N-pyrimidinyl- ou triazinylcarbamate de formule III

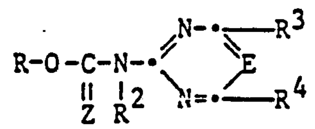

(III),

dans laquelle E, $R^2$, $R^3$, $R^4$ et Z ont la signification donnée pour la formule I et R est un phényle, alkyle ou phényle substitué, soit

b) on fait réagir un phénylsulfonylisocyanate ou isothiocyanate condensé de formule IV

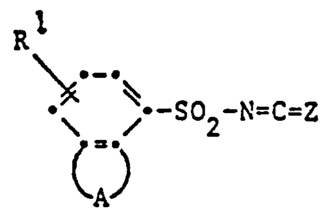

(IV),

dans lequel A, $R^1$ et Z ont la signification indiquée pour la formule I, en présence d'une base avec une aminopyrimidine ou -triazine de formule V

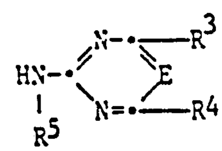

(V),

dans laquelle E, $R^2$, $R^3$ et $R^4$ ont la signification donnée pour la formule I soit

c) on fait réagir un N-phénylsulfonylcarbamate condensé de formule VI

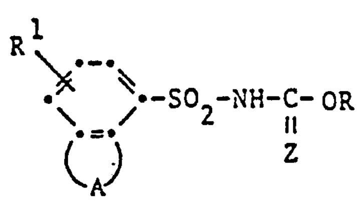

(VI),

dans laquelle A, $R^1$ et Z ont la signification donnée pour la formule I et R est un phényle, alkyle ou phényle substitué, avec une aminopyrimidine ou -triazine de formule V donnée ci-dessus, et transforme le cas échéant en les sels obtenus en faisant réagir une sulfonylurée de formule I avec une amine, un hydroxyde de métal alcalin ou alcalino-terreux ou une base d'ammonium quaternaire.

11. Utilisation de principes actifs de formule I selon la revendication 1 ou des moyens les contenant pour combattre la croissance indésirable de plantes.

12. Utilisation de principes actifs de formule I selon la revendication 1 ou des moyens les contenant pour bloquer la croissance de plantes.

13. Utilisation de principes actifs de formule I, selon la revendication 1, ou de moyens les contenant, pour influencer la croissance de plantes, dans le but d'augmenter le rendement.

14. Utilisation selon la revendication 11, pour combattre sélectivement avant ou après la levée, des mauvaises herbes dans les cultures de plantes vivrières.